(19) Europäisches Patentamt
European Patent Office
Office européen des brevets

(11) Publication number: **0 379 999 B1**

# (12) EUROPEAN PATENT SPECIFICATION

(45) Date of publication of patent specification: **21.12.94**

(51) Int. Cl.5: **C12P 19/04**, C08B 37/00, //(C12P19/04,C12R1:05)

(21) Application number: **90101119.7**

(22) Date of filing: **19.01.90**

The file contains technical information submitted after the application was filed and not included in this specification

## (54) Polysaccharide, and water absorbent, moisture absorbent or humectant and thickening agent chiefly made of the polysaccharide, and cultivation method of producing it by a microorganism.

(30) Priority: **19.01.89 JP 10398/89**
**08.01.90 JP 1359/90**

(43) Date of publication of application:
**01.08.90 Bulletin 90/31**

(45) Publication of the grant of the patent:
**21.12.94 Bulletin 94/51**

(84) Designated Contracting States:
**CH DE FR GB IT LI SE**

(56) References cited:

**AGRICULTURAL AND BIOLOGICAL CHEMISTRY, vol. 49, no. 8, 1985, Tokyo (JP); Y. HAYAKAWA et al., pp. 2443-2446&NUM;**

**CARBOHYDRATE RESEARCH, vol. 161, 1987, Elsevier Science Publishers B.V., Amsterdam (NL); T.A. CHOWDHURY et al., pp. 127-132&NUM;**

**CHEMICAL ABSTRACTS, vol. 96, no. 1, 04 January 1982, Columbus, OH (US); p. 460, no. 4951r&NUM;**

(73) Proprietor: **HAKUTO CORPORATION**
**1-13, Shinjuku 1-chome**
**Shinjuku-ku**
**Tokyo (JP)**

Proprietor: **AGENCY OF INDUSTRIAL SCIENCE AND TECHNOLOGY**
**3-1, 1-chome, Kasumigaseki**
**Chiyoda-ku**
**Tokyo (JP)**

(72) Inventor: **Kurane, Ryuichiro**
**253, Shinmatsudo 7-chome**
**Matsudo-shi, Chiba-ken (JP)**
Inventor: **Suzuki, Tomoo**
**5-1, Ottominami 1-chome**
**Tsuchiura-shi, Ibaraki-ken (JP)**
Inventor: **Nohata, Yasuhiro**
**C-1504 Shoseki Shataku, Midorigaoka**
**Yokkaichi-shi, Mie-ken (JP)**

(74) Representative: **Strehl Schübel-Hopf Groening & Partner**
**Maximilianstrasse 54**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**PURE AND APPLIED CHEMISTRY, vol. 56, no. 7, 1984, Pergamon Press Ltd., London (GB); P.A. SANDFORD et al., pp. 879-892&NUM;**

**Description**

The present invention relates to a microorganism-derived water absorbent, moisture absorbent or humectant and thickening agent, as well as a method of enhancing their production. The present invention is expected to find use in a broad range of applications including moisture absorbents, humectants and thickening agent such as sanitary articles and paper diapers, cosmetics and even covering humectants for water for irrigation of seedlings to be used in the greening of deserts.

With the recent modernization of life style, the consumption of sanitary articles and paper diapers is increasing year by year. Most of the water absorbents, moisture absorbents or humectants and thickening agent used in sanitary articles and paper diapers are based on synthetic high polymer materials. Since currently available sanitary articles and paper diaper are of a disposable type, they are washed in flush toilets and discharged into the environment. However, they are hardly biodegradable and will remain in the environment for a prolonged period, which is not only unseemly but also deleterious to the environment. Thus, the development of alternatives that are biodegradable and which hence are compatible with the environment is strongly desired.

With the recent advances in biotechnology, attempts have been made to incorporate biomaterials in cosmetics. However, the use of such "biocosmetics" is very limited and there is a growing need for the development of new organism-derived moisture absorbents or humectants and thickening agent that can be used as the base of cosmetics.

Accelerated expansion of deserts is currently a global concern of the ecology of the earth. Japan is making a contribution to the greening of deserts by supplying Egypt and other desert countries with synthetic high polymer water absorbents, moisture absorbents or humectants that are to be used to retain water for irrigating seedlings. If such water absorbents, moisture absorbents or humectants and thickening agent were organism-derived ones having biodegradability and compatibility with the environment, they would hardly cause adverse effects on the environment after the growth of seedlings.

It is an object of the present invention to provide a microorganism-produced polysaccharide consisting essentially of rhamnose, fucose, glucose, mannose and glucuronic acid which are present in a molar ratio of (1 - 10):(2 - 10):(4 - 20):(1):(1 - 5).

Another object of the present invention is to provide a water absorbent, moisture absorbent or humectant and thickening agent comprising a microorganism-produced polysaccharide that overcomes and eliminates the problems with conventional synthetic high polymer versions and which hence has high biodegradability and can be used without causing potential environmental hazards such as secondary pollution.

Another purpose of the present invention is to provide a method of producing such improved water absorbent, moisture absorbent or humectant and thickening agent by cultivating a microorganism.

Other objects and advantages of the present invention may become apparent to those skilled in the art from the description and the drawings.

Fig. 1 is a UV absorption spectrum of the biopolymer produced in accordance with the present invention. The vertical axis plots light absorbance and the horizontal axis plots wavelength (200 - 300 nm);

Fig. 2 is an IR absorption spectrum of the biopolymer produced in accordance with the present invention. The vertical axis plots light transmittance (%) and the horizontal axis plots wave number ($cm^{-1}$);

Fig. 3 is a diagram showing the results of three measurements conducted to determine the intrinsic viscosity ($\eta$ = 42) of the biopolymer produced in accordance with the present invention. The vertical axis plots intrinsic viscosity ($\eta$) and the horizontal axis plots the concentration of sample (ppm);

Fig. 4-A is a liquid chromatographic chart of standard samples (neutral sugars: glucose, mannose, rhamnose and fucose). The vertical axis plots dielectric constant ($\times 10^{-1}$ volts) and the horizontal axis plots retention time ($\times$ 10 minutes);

Fig. 4-B is a liquid chromatographic chart of the HCl hydrolyzate of the biopolymer produced in accordance with the present invention. The vertical and horizontal axes plot the same parameters as in Fig. 4-A;

Fig. 4-C is a diagram showing the relationship between the UV absorbance of a standard sample and retention time (minute);

Fig. 4-D is a diagram showing the relationship between the UV absorbance of the hydrolyzate of the biopolymer produced in accordance with the present invention and the retention time (minute);

Fig. 5-A shows a gas chromatographic pattern of trimethylsilylated derivatives of standard samples [rhamnose, fucose, uronic acid (glucuronic acid), mannose and glucose]. The vertical axis plots peak height and the horizontal axis plots retention time (minutes);

Fig. 5-B shows a gas chromatographic pattern of a trimethylsilylated derivative of the hydrolyzate of the biopolymer produced in accordance with the present invention. The vertical axis plots peak height and the horizontal axis plots retention time (minute);

Figs. 6-1A, 6-2A, 6-3A and 6-4A show mass spectra for rhamnose, fucose, glucose and mannose, respectively, of trimethylsilylated derivatives of their standard samples. The vertical axis plots intensity and the horizontal axis plots m/e;

Figs. 6-1B, 6-2B, 6-3B and 6-4B show mass spectra of rhamnose, fucose, glucose and mannose, respectively, of a trimethylsilylated derivative of the hydrolyzate of the biopolymer produced in accordance with the present invention. The vertical axis plots intensity and the horizontal axis plots m/e;

Fig. 7-A shows a gas chromatographic pattern of trimethylsilylated derivatives of standard sugars at specified concentrations. The vertical axis plots peak height and the horizontal axis plots retention time (minutes);

Fig. 7-B shows a gas chromatographic pattern of a trimethylsilylated derivative of the hydrolyzate of SP produced in Example 1. The vertical axis plots peak height and the horizontal axis plots retention time (minute);

Fig. 8 is a diagram showing the yield of production of a water absorbent, moisture absorbent or humectant and thickening agent when various carbon sources were used in cultivation of Alcaligenes latus strain B-16.

Fig. 9 is a graph showing the viscosity vs concentration characteristics of a biopolymer of the present invention ($SP_2$) and Kelzan (control), with the vertical and horizontal axes plotting viscosity (cps) and polymer concentration (w/w %), respectively;

Figs. 10-A and 10-B show fluidity curves for aqueous solutions of $SP_2$ and Kelzan, with the vertical and horizontal axes plotting viscosity (cps) and the rotational speed of spindle (rpm), respectively;

Fig. 11 is a graph showing the viscosity vs temperature characteristics of $SP_2$ and Kelzan (control), with the vertical and horizontal axes plotting viscosity (cps) and temperature (°C), respectively;

Fig. 12 is a graph showing the relationship between the concentration of NaCl in aqueous solutions of $SP_2$ and Kelzan (control) and their viscosity, with the vertical and horizontal axes plotting viscosity (cps) and NaCl concentration (w/w %), respectively; and

Fig. 13 is a graph showing the relationship between the pH of aqueous solutions of $SP_2$ and Kelzan (control) and their viscosity, and the vertical and horizontal axes plotting viscosity (cps) and pH, respectively.

Any organism can be used in the present invention as long as it belongs to the genus Alcaligenes and has the ability to produce in vivo a water absorbent, moisture absorbent or humectant and thickening agent. A representative example is Alcaligenes latus strain B-16 which has been deposited with the Fermentation Research Institute, the Agency of Industrial Science and Technology, under accession number FERM BP-2015.

The morphological and biochemical characteristics of this strain are shown in Table 1 below. Comparing these mycological properties with those described on page 372 of Bergey's Manual of Systematic Bacteriology, Volume 1, 1984, the present inventors found that the strain belongs to the genus Alcaligenes and is compatible with the environment. The type strain (ATCC 29712) is different from FERM BP-2015 in terms of the following aspects described in Table 1: deoxyribonuclease, citric acid, alginin dehydrase, acrylamidase, acid formation from sugars and the ability to produce extracellular polymers, but the two strains are identical in all other aspects.

Table 1

| Morphological and Biochemical Characteristics | |
|---|---|
| Properties | |
| Gram's stain | - |
| Morphology | short rod |
| Motility | + |
| Flagellum | peritrichous |
| Aerobic growth | + |
| Catalase | + |
| Oxidase | (+)* |
| OF test | 0 |
| Deoxyribonuclease | - |
| Nitric acid reduction | - |
| Citric acid | - |
| Alginin dehydrase | - |
| Acrylamidase | - |
| Acid formation from sugars | |
| glucose | + |
| maltose | + |
| xylose | + |
| fructose | + |
| sucrose | + |
| mannitol | - |
| Extracellular polymer producing ability | + |
| GC content (%) | 72 ± 1 |

* (+): weakly positive

Preferred carbon sources for the growth of this strain include not only monosaccharides and oligosaccharides such as fructose, glucose and sucrose but also natural polymers such as hemicellulose, starch and corn starch, and oils such as olive oil. Other medium constituents that can be used include inorganic nitrogen sources such as urea, ammonium chloride, ammonium nitrate and ammonium sulfate, organic nitrogen sources such as tryptone, yeast extract, meat extract, peptone and malt extract, and inorganic salts such as potassium phosphate, magnesium sulfate and sodium salt.

Using monosaccharides or disaccharides as carbon sources is particularly preferred for the purpose of producing a water absorbent, moisture absorbent or humectant. Cultivation with a phosphate added at a concentration of at least 80 mM is also desirable for enhanced production of these agents.

Cultivation may be liquid cultivation. The initial pH for cultivation ranges from 4 to 10, with the temperature adjusted to lie between 15 and 40 °C. Cultivation is usually performed with agitation under an aerated condition. The cultivation period which depends on carbon source and other factors will usually range from 1 to 10 days, during which a maximum production phase is set.

The culture as treated is a colorless and clear or pale yellow solid anionic polymer with a viscosity of about 1,000 - 15,000 mPas. Viscosity measurement is performed with a rotary viscometer after 100 volumes of water (20 °C) is added and completely absorbed by the treated culture.

By cultivation, a culture having a water absorbing, moisture absorbing or moisture retaining ability is obtained. The liquid culture is mixed with twice the volume of ethanol and the mixture is left to stand overnight at 5 °C. The resulting precipitate is collected by passage through No. 2 filter paper, washed three times with 70% ethanol, further washed three times with distilled water on filter paper, and dehydrated by freeze-drying or some other suitable method. In this way, a water absorbent, moisture absorbent or humectant can be recovered as a treated product of the culture. It should, however, be noted that the culture need not be treated by the isolation and purification procedures described above and if desired, it may be immediately put to use.

The water or moisture that can be absorbed or retained by the agent of the present invention is not limited to any particular type. It is generally held that the effectiveness of synthetic high polymer water absorbents, moisture absorbents or humectants is appreciably reduced in salt water compared to their

ability in pure water. However, as will be apparent from the examples that are given later in this specification, the organism-produced water absorbent, moisture absorbent or humectant agent of the present invention exhibits its intended effect even in the presence of salt and this may as well be considered a novel and outstanding feature over the conventional synthetic high polymer versions.

The methods by which the agent of the present invention absorbs water or moisture and retains moisture are in compliance with the standard assay methods described below for evaluating the performance of said agent. It should however be noted that the methods of implementing the present invention are by no means limited to these standard methods alone.

The capabilities of the agent of the present invention for absorbing water, absorbing moisture and retaining moisture were measured by the following methods.

(A) Water Absorbing Capability

A method generally referred to as a "tea bag method" was adopted. A container having a capacity of about 20 ml was made from a non-woven fabric ("Kitchen Tauper" of Tokai Pulp Co., Ltd.; 100% natural pulp) and charged with a predetermined weight of a sample such as a dried polymer. Then, the container was immersed in pure water for 2 hours, recovered and left to stand for 1 hour to remove the surplus water. The dehydrated sample was put into a constant weight beaker (10 ml) and its weight after water absorption (the weight of water absorbed + sample's weight) was measured exactly. Thereafter, the sample was dried at 105 °C for ca. 15 hours to completely evaporate the water. The exact weight of the sample was again measured.

After these measurements, the amount of water absorption (g) per gram of the dried sample was calculated by the following equation:

$$\text{Water absorption} = \frac{\text{sample weight after absorption (g)} - \text{sample weight before absorption (g)}}{\text{dried sample's weight (= sample weight before absorption) (g)}}$$

(B) Moisture Absorbing Capability

Measurements were conducted in accordance with the method described in Koshokaishi (J. of Perfume and Cosmetics), Vol. 8, No. 2, p. 131 (1984) as following. Desiccators which respectively contained a saturated solution of potassium nitrate (91% relative humidity, r.h.), a saturated solution of sodium nitrate (61.8% r.h.) and a saturated solution of magnesium chloride (31.9% r.h.) were used as stored in a thermostatic chamber at 37 °C. Dried samples were exactly weighed in an amount of about 100 mg in plastic cups (i.d. 1.2 cm; product of Sanplatec Corp.) and left to stand in the desiccators. After 2, 4, 6, 8 and 24 hours, the weights of the samples were measured and the percentages of moisture absorbed by these samples were determined by the following equation:

$$\text{Percentage of moisture absorption (\%)} = \frac{W_t - W_o}{W_o} \times 100$$

where $W_o$ is the sample weight before standing and $W_t$ is the sample weight measured at given intervals.

(C) Moisture Retaining (Water Holding) Capability

The method of measuring the moisture retaining capability is described in Koshokaishi, ibid. Desiccators which respectively contained a saturated solution of sodium nitrate (64.8% r.h.), a saturated solution of magnesium chloride (33% r.h.) and phosphorus pentoxide (34% r.h.) were used as stored in a thermostatic chamber at 20 °C. Additional desiccators which respectively contained a saturated solution of sodium nitrate (64.8% r.h.) and silica gel were also used as stored in a thermostatic chamber at 20 °C. Dried samples were exactly weighed in amounts of about 100 mg in plastic cups, mixed with 20 µl of water, exactly weighed again and left to stand in the desiccators. After the standing, the weights of the respective samples were

measured as in (B) and their ability to retain moisture was determined by the following equation, with the percentage of residual water being used as an index:

$$\text{Percentage of residual water (\%)} = (1 - \frac{W_o - W_t}{20}) \times 100$$

where $W_o$ is the weight of the hydrous sample before standing and $W_t$ is the weight of the hydrous sample measured at given intervals.

## Examples

The present invention is hereunder described in greater detail by way of Examples and Comparative Examples which are given here for illustrative purposes only and are by no means intended to limit the scope of the invention.

### Example 1

Production of Water Absorbent, Moisture Absorbent or Humectant Agent by Cultivation and Recovery of the Same :

Sucrose (15 g), $KH_2PO_4$ (6.8 g), $K_2HPO_4$ (8.8 g), $MgSO_4 \cdot 7H_2O$ (0.2 g), NaCl (0.1 g), urea (0.5 g) and meat extract (0.5 g) were dissolved in 1,000 ml of distilled water and the medium was adjusted to a pH of 7.4. A portion (150 ml) of the medium was transferred into a 500-ml conical flask and sterilized by autoclaving at 120 °C for 15 minutes. Thereafter, a loopful of Alcaligenes latus strain B-16 (FERM BP-2015) was inoculated into the medium in the flask and subjected to rotary shaking culture at 30 °C (180 rpm).

After 6 days of cultivation, a biopolymer was harvested and purified from the culture broth by the following procedure.

The culture (500 ml) was mixed with two volumes of ethanol and the mixture was left to stand. The liquid phase was removed from the mixture and the precipitate was recovered. To the recovered precipitate, 100 ml of pure water was added and the precipitate was dissolved by heating in a hot water bath at 60 - 70 °C. To the solution, five volumes of ethanol was added and the precipitate was recovered. By repeating the cycle of dissolution and precipitation several times, coloring materials that would have derived from the components of the culture medium were eliminated from the system and a white precipitate was obtained. The white precipitate obtained by the decoloring step described above was diluted and redissolved in 4000 - 8000 ml of a 0.02% NaOH solution, heated at 121 °C for 10 minutes and subjected to dilution and centrifugation at 40,000 g × 40 minutes in order to remove the cells. The cell-free supernatant was neutralized with hydrochloric acid and concentrated with a rotary evaporator at 60 - 70 °C. To the concentrate, 100 ml of pure water was added and the concentrate was redissolved by heating in a hot water bath at 60 - 70 °C. To the solution, five volumes of ethanol was added and a precipitate was obtained. After repeating the cycle of dissolution and precipitation with ethanol three times, the precipitate was vacuum-dried at ordinary temperatures to obtain a white purified biopolymer which was homogeneous by high-performance liquid chromatography.

By the purifying step described above, 2.4 - 3 g of a white purified biopolymer was obtained from 1,000 ml of the culture.

The purified biopolymer obtained in Example 1 had the following physicochemical properties.

(1) Color: white

(2) Carbonization temperature: 225 - 280 °C

(3) Elemental analysis:

Carbon and hydrogen contents were determined with a Carlo Erba C & H Analyzer (Carlo Erba S.P.A.) The oxygen content was calculated by subtracting the sum of C and H contents from 100 (wt%):

| | |
|---|---|
| C | 40 ± 4 |
| H | 6 ± 1 |
| O | 54 ± 5 |

(4) Solubility:

Slightly soluble in water (neutral); soluble in alkalies; insoluble in methanol, ethanol and acetone;

(5) UV absorption spectrum:

As shown in Fig. 1, no absorption was detected at 280 nm characteristic of proteins (peptides) and at 260 nm characteristic of nucleic acids.

(6) IR absorption spectrum:

As shown in Fig. 2, an absorption pattern characteristic of polysaccharides was observed near 800 - 1200 $cm^{-1}$; an absorption pattern characteristic of uronic acid was observed at 1620 ± 20 $cm^{-1}$; CH and $CH_2$ absorption patterns due to carbohydrates were observed near 2950 $cm^{-1}$; and a OH absorption pattern due to carbohydrates was observed near 3400 ± 20 $cm^{-1}$. These results suggest that the purified biopolymer of interest would be an acidic polysaccharide chiefly composed of sugars and other carbohydrates.

(7) Viscosity:

The viscosity of the biopolymer was measured with a Ubbelohde's viscometer with 0.1 N $NaNO_3$ being used as a solvent. The results of measurement are shown in Fig. 3. This biopolymer was found to have an intrinsic viscosity ($\eta$) of 42.

(8) Optical rotation:

The biopolymer of interest (100 ppm) was dissolved in a 0.02% NaOH solution and the solution was filtered through a 0.45 $\mu$m Millipore filter. The optical rotation of the filtrate was measured with a polarimeter (Model DIP 360 of Japan Spectroscopic Co., Ltd. with 100 mm of a standard cell). The biopolymer was found to have an optical rotation ($\alpha$) of 0.002 deg.

(9) Qualitative and quantitative reactions on sugars:

Cultivation was performed as in Example 1 using sucrose or fructose as a carbon source. The cultures were purified and the purified samples obtained were designated SP and FP, respectively. Each sample was subjected to qualitative and quantitative reactions on sugars. In the anthrone reaction and the phenol sulfate procedure, the results were evaluated in terms of glucose. In the Elson-Morgan method, hexosamines (e.g. glucosamine and galactosamine) were used as indices; in the periodic acid-resorcinol reaction, sialic acids (e.g. N-acetylneuraminic acid and N-glycolylneuraminic acid) were used as indices; in the carbazole sulfate reaction, uronic acids (e.g. glucuronic acid and galacturonic acid) were used as indices; and in the orthocin $Fe^{3+}$ method, glucuronic acid was used as an index. The biopolymers of interest were hydrolyzed by the following scheme. The results of reactions performed on the respective samples are summarized in Table 2.

Method of Hydrolyzing Polysaccharides

heat in 2 N $H_2SO_4$ at 100°C for 2 hours (sealed under vacuum)
↓
neutralize with $Ba(OH)_2$
↓
centrifuge at 18,000 rpm for 5 minutes to remove the precipitate
↓
stir with activated carbon for 5 minutes
↓
centrifuge at 19,000 rpm for 5 minutes to remove the precipitate
↓
filter through 0.45-μm membrane
↓
concentrate with evaporator at 50°C

Table 2

| Qualitative and Quantitative Reactions on Constituent Sugars | | |
|---|---|---|
| Reaction | Sugar content in each sample | |
| | SP | FP |
| Anthrone reaction | 76% | 66% |
| Phenol sulfate procedure | 78% | 58% |
| Elson-Morgan | - | - |
| Periodic acid-resorcinol reaction | - | - |
| Carbazole sulfate reaction | 19% | 17% |
| Orthocin $FE^{3+}$ reaction | 4% | 3% |

In the anthrone reaction and the phenol sulfate procedure, the results were expressed in percentages in terms of glucose.

The results of the qualitative and quantitative reactions on sugars suggest the possibility that the biopolymers of interest have hexose and uronic acids as constituents. It is however clear that the biopolymers do not have hexosamines such as glucosamine or sialic acids such as N-acetylneuraminic acid.

(10) Constituent sugars:

Now that it was established that the biopolymers of interest had sugars such as hexose and uronic acids, they were hydrolyzed with an acid such as hydrochloric acid and subjected to identification of constituent sugars by thin-layer chromatography, liquid chromatography, gas chromatography and mass spectroscopy.

(A) Thin-Layer Chromatography

Cultivation was performed as in Example 1 using sucrose as a carbon source and the culture was purified to obtain sample SP. A hydrolyzate of this sample was subjected to thin-layer chromatography under various developing conditions including solvent system. Rf values of known sugars and the hydrolyzate of the sample as obtained for various developing solvents are summarized in Table 3-1

(comparison of Rf values between each standard sugar and the hydrolyzate of the sample), Table 3-2 (comparison with mannose) and Table 3-3 (comparison with glucuronic acid as uronic acid).

The following conditions were used to identify constituent sugars by thin-layer chromatographic analysis.

Identification of Sugars by TLC

Experimental conditions:

1. TLC plate
   α. Kiesel Gel 60 of Merck
   β. Silica Gel 60A of Whatman
2. Developing temperature 50 °C
3. Color producing agent
   i) diphenylamine-aniline-phosphate reagent
   ii) naphthoresorcinol phosphate reagent
   iii) potassium permanganate reagent
4. Developing solvent
   a) l-propanol/water = 85/15
   b) ethyl acetate/acetic acid/methanol/water = 60/15/15/10
   c) t-butanol/acetone/0.1 M lactic acid = 4/4/2
   d) isopropanol/acetone/0.1 M lactic acid = 4/4/2
   e) acetone/0.1 M lactic acid/ethyl acetate = 6/2/2
   f) t-butanol/acetone/0.1 M lactic acid = 6/2/2
   g) isopropanol/0.1 M lactic acid/methanol = 4/2/4
   h) ethyl acetate/acetic acid/methanol/0.1 M lactic acid = 60/10/25/5
5. Preconditioner of TLC plate
   A. 0.1 M $NaHSO_3$
   B. 0.5 M $NaH_2SO_4$
6. Unless otherwise noted, only one development was done.

Table 3-1

| Rf Values in Thin-Layer Chromatography of Standard Sugars and Hydrolyzates of the Sample | | |
|---|---|---|
| | SP | Rf |
| | plate α, color producing reagent i, solvent c, preconditioner B, developed twice | plate β, color producing reagent i, solvent d, preconditioner B, developed once |
| Sample | 0.05, 0.37, 0.45, 0.57, 0.78, 0.88, 0.92, 0.95 | 0.40, 0.52, 0.79, 0.94 |
| D-glucose | 0.33 | 0.40 |
| D-galactose | 0.21 | 0.22 |
| D-mannose | 0.43 | 0.49 |
| D-xylose | 0.55 | 0.65 |
| L-arabinose | 0.50 | 0.46 |
| D-ribose | 0.66 | |
| L-ribose | 0.42 | |
| L-fucose | 0.78 | 0.77 |
| L-rhamnose | 0.91 | 0.92 |
| 2DOX-D-glucose | 0.84 | 0.88 |
| 2DOX-D-ribose | 0.94 | |
| D-glucuronic acid | 0.76, 0.79, 0.83, 0.85, 0.95 | 0.94 |
| D-galacturonic acid | 0.19 | |
| D-galactosamine | 0.03, 0.55, 0.70, 0.83 | |
| D-trehalose | 0.18 | |
| Maltose | 0.19 | 0.24 |
| D-lactose | 0.13, 0.23 | 0.15 |
| D-cellobiose | 0.21, 0.94 | |
| Melibiose monohydrate | 0.07 | |
| Methyl-α-D-glucopyranoside | 0.87 | |
| Salicin | 1.0 | |
| Raffinose | 0.06 | |
| Gulose | | 0.48 |
| Allose | | 0.42 |
| Talose | | 0.51 |

Table 3-2

Separation and Determination of Hexose (mannose)

Experimental conditions: α, ii, g, B

| | Rf |
|---|---|
| Sample | 0.14, 0.20, 0.37, 0.56 |
| Mannose | 0.19 |
| Talose | 0.22 |
| Talose + sample | 0.16, 0.21, 0.26, 0.40, 0.60 |

Table 3-3

Separation and Determination of Uronic Acids

(1) Experimental conditions: α, i, g, B

| | Rf |
|---|---|
| Sample | 0.23, 0.73, 0.89, 0.94 |
| Galacturonic acid | 0.09 |
| Glucuronic acid | 0.17 |
| Mixture of the above | 0.1, 0.21 |

(2) Experimental conditions: α, ii, e, A

| | Rf |
|---|---|
| Sample | 0.11, 0.5, 0.67, 0.77 |
| Muramic acid | 0.16 |
| Mannuronic acid lactone | 0.73 |

(3) Experimental conditions: α, iii, b, A

| | Rf |
|---|---|
| Sample | 0.11, 0.26, 0.43, 0.64, 0.83 |
| Glucorono lactone | 0.26, 0.51, 0.93 |
| Gulorono lactone | 0.07, 0.31, 0.43, 0.57, 0.67 |

Table 3-4

| Rf Values in Thin-Layer Chromatography of Hydrolyzates of the Sample and a Mixture of Five Standard Samples (glucose, mannose, rhamnose, fucose and glucuronic acid) | | |
|---|---|---|
| Condition | Sample (hydrolyzate) | Mixture (of standard sample) |
| α, i, a, A developed twice | 0.11, 0.2, 0.58, 0.68, 0.75, 0.82 | 0.14, 0.56, 0.61, 0.73, 0.80 |
| α, i, b, A developed twice | 0.36, 0.42, 0.53 0.68, 0.80, 0.85 | 0.24, 0.31, 0.39, 0.52 0.71, 0.81, 0.85 |
| α, i, c, B developed twice | 0.33, 0.5, 0.73, 0.91, 0.95 | 0.38, 0.5, 0.74, 0.9, 0.95 |
| β, i, d, B | 0.41, 0.54, 0.78, 0.95, 0.98 | 0.40, 0.52, 0.77, 0.94, 0.98 |

The data in Table 3-1 shows that the biopolymer of interest contains glucose, rhamnose and fucose since it has Rf values in agreement with those of glucose, rhamnose and fucose, as standard.

As shown in Table 3-2, talose had an Rf value similar to that of mannose. Thus, talose was compared with the hydrolyzate of the sample under various conditions. As a result, it was found that the sample biopolymer contained mannose rather than talose as a constituent sugar.

In order to make further sure that glucuronic acid was a constituent uronic acid, an experiment was conducted under the three conditions shown in Table 3-3. As shown under Experimental Conditions (1), glucuronic acid alone had an Rf value of 0.17 but in the presence of galacturonic acid, even the standard sample had a higher Rf value of 0.21. The experiment conducted under the other conditions showed that the uronic acid was neither muramic acid nor mannuronic acid lactone. It therefore became apparent that the biopolymer of interest contained glucuronic acid as a uronic acid.

With these results taken into consideration, five standard samples (glucose, mannose, rhamnose, fucose and glucuronic acid) in admixture and the hydrolyzate of the biopolymer of interest were subjected to thin-layer chromatography under four conditions (see Table 3-4). As shown in Table 3-4, the five standard samples had Rf values which were in very good agreement with those of the hydrolyzate of the biopolymer.

Thus, the results of detailed analyses by thin-layer chromatography showed that the biopolymer of interest contained five sugars, glucose, mannose, rhamnose, fucose and glucuronic acid, as constituents.

(B) High-Performance Liquid Chromatography

The neutral sugars (rhamnose, fucose, mannose and glucose) identified by thin-layer chromatography were analyzed with a high-performance liquid chromatograph using Amide-80 (Tosoh Corp.) as a column: mobile phase, acetonitrile/water = 80/20; flow rate, 1.0 ml/min.; column temperature, 80 °C; detector, RI. Liquid chromatographic charts of the standard samples of the neutral sugars and the hydrolyzate of the biopolymer sample prepared in Example 1 are shown in Fig. 4-A and Fig. 4-B, respectively. As shown in these figures, the hydrolyzate of the biopolymer sample of interest had peaks 1, 2, 3 and 4 which respectively corresponded to rhamnose, fucose, mannose and glucose.

A standard sample of glucuronic acid and the hydrolyzate of the biopolymer were compared by high-performance liquid chromatography using a phenol-form column (Waters, Inc.): mobile phase, methanol/water = 80/20; flow rate, 0.5 ml/min.; detector, UV. The results are shown in Fig. 4-C and Fig. 4-D, from which one can see that the two samples had the same retention time.

The above results show that the biopolymer of interest as analyzed by high-performance liquid chromatography (HPLC) contained glucose, mannose, rhamnose, fucose and glucuronic acid as constituent sugars.

(C) Gas Chromatography and Gas Mass Spectroscopy

In order to perform a triple check of the constituent sugars identified by TLC and HPLC, the biopolymer of interest was subjected to gas chromatography and gas mass spectroscopy (GC-MS). Simultaneous analysis of neutral sugars and uronic acid (glucuronic acid) was performed by the following procedure: the sample obtained in Example 1 was hydrolyzed with HCl, silylated with a silylating agent, loaded into a gas chromatographic column (supported by Silicone OV-l), heated to a temperature in the range of 50 - 200 °C, and analyzed by FID. Hydrolysis of the sample was conducted in accordance with the method of hydrolyzing polysaccharides as described on page 13. The hydrolyzate was derivated to a trimethylsilyl form by the following scheme.

Derivation to Trimethylsilyl

```
Hydrolyzate
   |
   |--20 µl of ethanethiol/trifluoroacetic acid
   |  (2/1, v/v)
   |  (Mercaptalation)
   |
   25°C × 10 minutes (in closed test tube with a screw
   cap)
   |
   |--50 µl of pyridine, 100 µl of HMDS, 50 µl of TMCS
   |  (Trimethylsilylation)
   |
   50°C × 30 minutes (with occasional stirring)
   |
Centrifuge
   |
 -------------
 |           |
ppt         sup
            (GLC analysis)
            internal standard:  3-methyl-D-glucose
```

A gas chromatographic pattern of the authentic samples of trimethylsilylated derivatives of glucose, mannose, rhamnose, fucose and uronic acid (glucuronic acid) is shown in Fig. 5-A. A gas chromatographic pattern of the hydrolyzate of the purified biopolymer obtained in Example 1 is shown in Fig. 5-B. As these figures show, the silylated derivative of the hydrolyzate of the sample of interest was in complete agreement with the silylated derivatives of glucose, mannose, rhamnose, fucose and glucuronic acid.

Four peaks that were comparatively large in the gas chromatographic analysis (peak 1, rhamnose; peak 2, fucose; peak 5, glucose; peak 6, mannose) were introduced into a mass spectrograph and subjected to GC-MS analysis.

Fig. 6-1A and Fig. 6-1B show mass spectra of peak 1 and rhamnose of the standard samples and the hydrolyzate of the biopolymer, respectively; Fig. 6-2A and Fig. 6-2B show mass spectra of peak 2 and fucose of the standard samples and the hydrolyzate, respectively; Fig. 6-3A and Fig. 6-3B show mass spectra of peak 5 and glucose of the standard samples and the hydrolyzate, respectively; and Fig. 6-4A and Fig. 6-4B show mass spectra of peak 6 and mannose of the standard samples and the hydrolyzate, respectively. As these mass spectra show, the fragments of peaks 1, 2, 5 and 6 are in agreement with those of the standard samples.

Thus, the results of GC-MS analysis also confirm that the hydrolyzate of the biopolymer of interest contained rhamnose, fucose, mannose and glucose as constituent sugars.

In summary, the results of gas chromatography (GC) and gas mass spectroscopy (GC-MS) showed that the hydrolyzate of the biopolymer of interest contained glucose, mannose, rhamnose, fucose and glucuronic acid as constituent sugars.

(11) Molar ratio of constituent sugars:

The molar ratio of the five constituent sugars, rhamnose, fucose, mannose, glucose and glucuronic acid, was determined from the ratio of areas of individual peaks in gas chromatography. The conditions of gas chromatography were the same as those described on pages 19 - 20. In order to determine the molar ratio of the constituent sugars, the standard samples of specified concentrations were first subjected to gas chromatography and the areas of peaks obtained were determined. Then, the hydrolyzate of the purified biopolymer obtained in Example 1 (for the conditions of hydrolysis, see page 13) was subjected to gas chromatography and the areas of peaks obtained were determined. On the basis of the thus determined peak area, the molar ratio of the constituent sugars under consideration was calculated by the following equation:

$$\text{Molar ratio of constituent sugar} = \frac{\text{Peak area of hydrolyzate (constituent sugar)}}{\text{Peak area of standard sample}} \times (\text{Number of moles of standard sample})$$

The gas chromatographic pattern of the standard samples used is shown in Fig. 7-A, and that of the hydrolyzate of SP (purified biopolymer obtained by cultivation in Example 1 with sucrose used as a carbon source) is shown in Fig. 7-B. The peak areas and the number of moles of the standard samples and the hydrolyzate are shown in Table 4 below.

Table 4

| | Standard sample | | SP hydrolyzate | |
|---|---|---|---|---|
| | Peak area | Number of moles (mmol) | Peak area | Number of moles (mmol) |
| Rhamnose (peak 1) | 14884 | 2.44 | 5196 | 0.853 |
| Fucose (peak 2) | 11037 | 3.05 | 5675 | 1.568 |
| Glucose (peak 5) | 15574 | 2.78 | 17378 | 3.102 |
| Mannose (peak 6) | 13243 | 2.78 | 2730 | 0.573 |
| Glucuronic acid (peaks 3 + 4) | 9331 | 4.12 | 3218 | 1.421 |

The molar ratio of the constituent sugars may be calculated as follows with mannose taken as unity:
Rhamnose:fucose:glucose:mannose:glucuronic acid ≃ (1 - 2):(3 - 4):(5 - 6):(1):(2 - 3).

Example 2

Effects of Variations in the Type and Concentration of Carbon Sources and the Concentration of Phosphates on the Efficiency of Production of Water Absorbent, Moisture Absorbent or Humectant and Thickening Agent:

A water absorbent, moisture absorbent or humectant and thickening agent was produced under different culture conditions by changing the type and concentration of carbon sources and the concentration of phosphates as shown in Table 5. The other conditions were the same as in Example 1.

Table 5

| Symbol for culture conditions | Carbon source | | Phosphate | |
|---|---|---|---|---|
| | type | amount (g) | $K_2HPO_4$ (g) | $KH_2PO_4$ (g) |
| $SP_1$ | sucrose | 15 | 8.4 | 4.4 |
| $SP_2$ | sucrose | 15 | 16.8 | 8.8 |
| $FP_1$ | fructose | 15 | 8.4 | 4.4 |
| $FP_2$ | fructose | 15 | 16.8 | 8.8 |
| 1/5 $SP_1$ | sucrose | 3 | 1.7 | 0.9 |
| 1/5 $SP_2$ | sucrose | 3 | 33.6 | 1.8 |
| 1/5 $FP_1$ | fructose | 3 | 1.7 | 0.9 |
| 1/5 $FP_2$ | fructose | 3 | 33.6 | 1.8 |

Example 3

Water Absorbing Ability:

The water absorbing ability of the samples obtained in Example 2 was measured by the method described in (A). The results are shown in Table 7. The six controls or comparative samples described in Table 6 below were also tested.

Table 6

| Comparative sample | Manufacturer | Remarks |
|---|---|---|
| (1) Pulp | | |
| (2) Silica gel | Kanto Chemicals Co., Ltd. | reagent |
| (3) Ion-exchange resin | Dow Chemical | |
| (4) High-grade water-absorbing polymer | Sumitomo Chemical | Sumika Gel S-50 |
| (5) PVA | Unitika Kasei | UP-100G, 8-10 cps, partially saponified |
| (6) Anionic polymer | Sumitomo Chemical | Sumifloc FA-70 (acrylamide/acrylic acid copolymer; $\overline{MW}$, $7 \times 10^6$) |

It is obvious from Table 7 that the substances produced by Alcaligenes latus absorbed more water at a faster rate than any of the samples in the control group.

Table 7

| Water Absorption by Biopolymers | | |
|---|---|---|
| | Sample | Water absorption (g) per gram of dried sample |
| Test group | $SP_1$ | 759.6 |
| | $SP_2$ | 593.3 |
| | $FP_1$ | 451.6 |
| | $FP_2$ | 759.6 |
| | 1/5 $SP_1$ | 1349.0 |
| | 1/5 $SP_2$ | 836.8 |
| | 1/5 $FP_1$ | 1295.4 |
| | 1/5 $FP_2$ | 944.5 |
| Control group | Pulp | 3.8 |
| | Silica gel | 1.4 |
| | Ion-exchange resin | 2.5 |
| | High-grade water-absorbing polymer | 249.4 |
| | PVA | 4.6 |
| | Anionic polymer | 363.6 |

Example 4

Moisture Absorbing Ability:

The moisture absorbing ability of sample $SP_2$ prepared in Example 2 and a new sample MIX which was prepared by mixing all the samples prepared in Example 2 was measured by the method described in (B). The controls compared with $SP_2$ and MIX were the following common moisture absorbents: silica gel; PVP (polyvinylpyrrolidone sold from Wako Chemicals Industries, Ltd. under the trade name K-30); urea (guaranteed reagent available from Kanto Chemicals Co., Ltd.); glycerin (guaranteed reagent available from

Kanto Chemicals Co., Ltd.); PEG 200 (Nippon Yushi); and anionic polymer (Sumifloc FA-70 of Sumitomo Chemical). The test results are shown in Table 8 below.

Table 8

| Relative humidity | Sample | Moisture absorption (%) at given intervals (h) | | | | | | |
|---|---|---|---|---|---|---|---|---|
| | | 2 | 4 | 6 | 8 | 10 | 24 | 48 |
| 91% | $SP_2$ | 42 | 53 | 61 | 65 | 69 | 93 | 105 |
| | MIX | 38 | 50 | 58 | 62 | 66 | 87 | 99 |
| | silica gel | 31 | 32 | 32 | 32 | 32 | 31 | 31 |
| | PVP | 29 | 34 | 36 | 37 | 39 | 46 | 48 |
| | Urea | 12 | 23 | 35 | 45 | 57 | 114 | 154 |
| | PVA | 8 | 11 | 13 | 13 | 14 | 15 | 16 |
| | glycerin | 33 | 49 | 61 | 68 | 76 | 113 | 140 |
| | PEG 200 | 27 | 38 | 45 | 50 | 55 | 78 | 92 |
| | anionic polymer | 17 | 28 | 35 | 41 | 46 | 64 | 75 |
| | hyaluronic acid | | | | | | 35 | |
| 61.8% | $SP_2$ | 31 | 33 | 34 | 33 | 34 | 33 | 35 |
| | MIX | 28 | 30 | 31 | 32 | 32 | 31 | 30 |
| | silica gel | 28 | 29 | 29 | 29 | 29 | 29 | 29 |
| | PVP | 18 | 19 | 19 | 19 | 19 | 18 | 19 |
| | Urea | 0 | 0 | 0 | 0 | 0 | 0 | 0 |
| | PVA | 3 | 3 | 4 | 5 | 5 | 7 | 7 |
| | glycerin | 23 | 30 | 33 | 35 | 36 | 37 | 38 |
| | PEG 200 | 18 | 20 | 20 | 20 | 20 | 20 | 22 |
| | anionic polymer | 9 | 13 | 16 | 21 | 21 | 22 | 21 |
| | hyaluronic acid | | | | | | 17 | |
| 31.9% | $SP_2$ | 7 | 9 | 6 | 11 | 12 | 12 | 11 |
| | MIX | 9 | 10 | 7 | 14 | 14 | 14 | 14 |
| | silica gel | 13 | 16 | 12 | 18 | 18 | 17 | 16 |
| | PVP | 8 | 9 | 9 | 9 | 9 | 9 | 10 |
| | Urea | 0 | 0 | 0 | 0 | 0 | 0 | 0 |
| | PVA | 0 | 1 | 1 | 1 | 2 | 2 | 2 |
| | glycerin | 6 | 9 | 11 | 12 | 13 | 12 | 12 |
| | PEG 200 | 3 | 7 | 7 | 7 | 7 | 6 | 7 |
| | anionic polymer | 1 | 1 | 1 | 2 | 1 | 3 | 4 |

The data for glycerin and urea were substantially equal to the 24-hour values obtained by the method of (B) described in T. Ando et al., Koshokaisha, ibid, pp. 130 - 134. Hyaluronic acid has recently come to be used in cosmetics as naturally occurring moisture absorbent or humectant and as compared to its 24-hour values described in Ando et al. (ca. 35% at 91% r.h. and 37°C and ca. 17% at 61.8% r.h. and 37°C), MIX was from 2.7 to about 2.0 times more effective in absorbing moisture.

Thus, it is clear that $SP_2$ and MIX which are organism-derived biopolymers exhibit high moisture absorbing ability.

Example 5

Moisture Retaining (Water Holding) Ability:

$SP_2$ and MIX tested in Example 4 were also evaluated for their ability to retain moisture by the method described in (C). The controls were the same as those selected in Example 4. The test results are shown in Table 9.

According to Ando et al., ibid, p. 133, the 24-hour moisture retaining ability of hyaluronic acid (105%) was lower than the values for $SP_2$ and MIX both of which were at least 1.5 times as effective as hyaluronic acid.

By comparing the data in Table 9 with documented values, one will readily see that $SP_2$ and MIX which are organism-derived biopolymers exhibit high moisture retaining ability.

Table 9

| Relative humidity | Sample | Moisture absorption (%) at given intervals (h) | | | | | | |
|---|---|---|---|---|---|---|---|---|
| | | 2 | 4 | 6 | 8 | 10 | 24 | 48 |
| 64.8% | $SP_2$ | 155 | 159 | 162 | 163 | 168 | 167 | 162 |
| | MIX | 160 | 161 | 164 | 167 | 172 | 171 | 167 |
| | silica gel | 133 | 134 | 133 | 133 | 134 | 132 | 131 |
| | PVP | 132 | 118 | 118 | 117 | 120 | 120 | 115 |
| | Urea | 6 | 3 | 2 | 2 | 1 | 0 | 2 |
| | PVA | 61 | 32 | 30 | 30 | 31 | 31 | 33 |
| | glycerin | 156 | 178 | 189 | 195 | 203 | 207 | 200 |
| | PEG 200 | 140 | 141 | 144 | 146 | 150 | 151 | 143 |
| | anionic polymer | 96 | 99 | 103 | 105 | 111 | 111 | 105 |
| | hyaluronic acid | | | | | | 105 | |
| 33% | $SP_2$ | 100 | 99 | 96 | 95 | 97 | 84 | 82 |
| | MIX | 97 | 94 | 89 | 88 | 90 | 76 | 74 |
| | silica gel | 93 | 94 | 91 | 93 | 94 | 87 | 84 |
| | PVP | 77 | 73 | 70 | 69 | 70 | 63 | 60 |
| | Urea | 5 | 2 | 3 | 3 | 5 | 4 | 6 |
| | PVA | 30 | 27 | 25 | 25 | 25 | 22 | 21 |
| | glycerin | 79 | 76 | 72 | 72 | 74 | 58 | 55 |
| | PEG 200 | 66 | 61 | 57 | 56 | 58 | 46 | 43 |
| | anionic polymer | 43 | 37 | 36 | 35 | 36 | 33 | 33 |
| 34% (measured) under $P_2O_5$ | $SP_2$ | 51 | 32 | 24 | 26 | 24 | 26 | 9 |
| | MIX | 56 | 40 | 36 | 34 | 34 | 26 | 17 |
| | silica gel | 53 | 35 | 28 | 25 | 30 | 29 | 9 |
| | PVP | 38 | 24 | 26 | 23 | 20 | 11 | 6 |
| | Urea | -2 | -2 | -2 | -2 | -2 | -2 | -4 |
| | PVA | 37 | 25 | 22 | 20 | 19 | 14 | 9 |
| | glycerin | 39 | 24 | 20 | 18 | 16 | 4 | -2 |
| | PEG 200 | 23 | 12 | 15 | 19 | 11 | 14 | -31 |
| | anionic polymer | 30 | 25 | 22 | 21 | 20 | 16 | 10 |

Example 6

Water Absorption Test in the Presence of Salt:

It is generally held that the water absorption by synthetic high polymer water absorbents reduces markedly in the presence of salt. Thus, a test was conducted in order to see what would happen to the organism-produced water absorbent, moisture absorbent or humectant of the present invention when it was used in the presence of salt. The sample used in this test was $SP_2$ prepared in Example 2. Sodium chloride was added as a salt in an amount of 0.9%. Before being used in the test, the sample was pulverized frozen and further dried. To insure that the powder would absorb a maximum amount of water, the absorption time was extended from the normal 2 hours to 24 hours.

The control was a commercial high-grade water-absorbing synthetic polymer (Sumika Gel S-50), which was tested under the same conditions as described above. The test results are shown in Table 10 below.

Table 10

| Sample | Water absorbed | Water absorption (g) per gram of dried sample |
|---|---|---|
| $SP_2$ | pure water<br>0.9% physiological saline | 636 (24 hours)<br>384 (24 hours) |
| control (synthetic high polymer absorbent) | pure water<br>0.9% physiological saline | 200.9 (24 hours)<br>23.8 (24 hours) |

As Table 7 shows, the water absorbing ability of $SP_2$ certainly reduced in the presence of salt but the decrease was much smaller than what occurred in the control. Although the difference in absorption time precludes a definite conclusion, one may as well say that the water absorption by $SP_2$ in the presence of 0.9% NaCl is comparable to the 2-hour values for Sumika Gel S-50 and Sumifloc FA-70 in pure water which are shown in Table 7 (Example 3).

Thus, it is clear that $SP_2$ works satisfactorily as a water absorbent even if it is used in salt water.

Example 7

Production of Water Absorbent, Moisture Absorbent or Humectant and Thickening Agent in the Presence of Various Carbon Sources:

Cultivation was performed as in Example 1 except that the following four carbon sources were individually used at a concentration of 15 g/ℓ: fructose as a representative monosaccharide; sucrose as a representative disaccharide; starch as a representative natural polymer; and olive oil as a representative nonaqueous carbon source. A water absorbent, moisture absorbent or humectant and thickening agent was removed from the respective 6-day cultures and their weight on a dry basis was measured to compare the yields of the respective products. The results are shown in Fig. 8, from which one can see that the yield of the desired water absorbent, moisture absorbent or humectant and thickening agent could be increased by selective use of a monosaccharide or disaccharide as a carbon source in culture medium.

Example 8

Relationship between NaCl Concentration and Water Absorption:

In consideration of possible applications of the polysaccharide of the present invention such as paper diapers and sanitary napkins, the retention of its water absorbing capability in the presence of sodium chloride is a very important factor for its industrial and commercial use. Synthetic high polymer absorbents in current use are capable of absorbing pure (distilled) water 200 - 300 times their own weight but their ability is reduced to only 50 - 80 times their weight in the presence of physiological saline (0.9%). Thus, in order to verify the effectiveness of the polysaccharide of the present invention as a water absorbent, its water absorbing capability was investigated at varying NaCl concentrations and the results are shown in Table 11 below.

Table 11

| Relationship between NaCl Concentrations of Water Absorption | |
|---|---|
| NaCl concentration (%) | Water absorption (w/w) |
| 0 | 1439 |
| 0.25 | 612 |
| 0.5 | 579 |
| 1 | 452 |
| 2.5 | 376 |

As Table 11 shows, the polysaccharide of the present invention was capable of absorbing water 450 and 370 times its own weight at NaCl concentrations of 1% and 2.5%, respectively. These values were even higher than the ability of conventional superabsorbent polymers to absorb distilled water. This feature of the polysaccharide will prove to be a great advantage for its commercial use.

Example 9

The viscosity vs concentration characteristics of the powder of polymer sample $SP_2$ prepared in Example 2 were measured. Kelzan (general-purpose xanthan gum of Kelco Co.) all particles of which passed a 28-mesh screen was used as a control. Each of the sample and control was dissolved in pure water at a concentration of 1% (w/w) and thereafter diluted with pure water at varying concentration from 0.1 to 1%. The viscosity of the respective dilutions was measured with a Brookfield type viscometer (25 °C and 30 rpm on No. 2 spindle). The results are shown in Fig. 9, from which one can see that the sample of the present invention had higher viscosities than Kelzan at all concentrations tested.

Example 10

The physical characteristics of $SP_2$ and Kelzan in aqueous solution were measured. An aqueous solution containing 2000 ppm of $SP_2$ and an aqueous solution containing 5000 ppm of Kelzan were prepared. The rotational speed of No. 2 spindle in a Brookfield viscosmeter (pH 7.2 and 25 °C) was varied between 6 rpm and 60 rpm and the resulting changes in the viscosity of each solution was measured. The result for $SP_2$ is shown in Fig. 10-A and that for Kelzan is shown in Fig. 10-B, from which one can see that Kelzan displayed pseudoplastic characteristics. When viscosity measurements were conducted with increasing shear rate, $SP_2$ also displayed pseudoplastic characteristics as evidenced by decreasing viscosity. When the shear rate was lowered immediately after it reached a peak, the viscosity was lower than the previous value. When shearing was stopped and the solution was left to stand, its viscosity returned to the normal level. Because of this hysteresis in viscosity $SP_2$ was found to have thixotropic characteristics.

Example 11

The viscosity vs temperature characteristics of $SP_2$ were measured. An aqueous solution containing 2000 ppm of $SP_2$ and an aqueous solution containing 5000 ppm of Kelzan were prepared and their viscosity was measured with a Brookfield viscometer (pH 7.2; rotational speed of No. 2 spindle, 30 rpm) with temperature raised from 5 to 80 °C. The results are shown in Fig. 11, from which one can see that the viscosity of $SP_2$ was substantially constant irrespective of temperature although the viscosity of Kelzan decreased with temperature.

The viscosity of many synthetic and natural polymers increases with decreasing temperature and decreases with increasing temperature. Kelzan is one of the polymers known to experience minimum changes in viscosity with temperature but as is clear from Fig. 11, the viscosity change was much smaller in $SP_2$ than in Kelzan.

Example 12

The relationship between the NaCl concentration of an aqueous solution containing $SP_2$ and its viscosity was measured. Sodium chloride was dissolved in pure water at concentrations of 0.01%, 0.05%, 0.1%, 0.5% and 1.0% and 2000 ppm of $SP_2$ or 5000 ppm of Kelzan was dissolved in each of the resulting

NaCl solutions. Viscosity measurements were conducted with a Brookfield viscometer (25°C and pH 7.2) with No. 2 spindle rotated at 30 rpm. The results are shown in Fig. 12, from which one can see that the viscosity of $SP_2$ was less sensitive to NaCl concentration than that of Kelzan.

Example 13

The viscosity vs pH characteristics of $SP_2$ were measured. An aqueous solution of 0.1% NaCl was prepared and 2000 ppm of $SP_2$ or 5000 ppm of Kelzan was dissolved in this aqueous NaCl solution. After pH adjustment with HCl or NaOH, viscosity measurements were conducted with a Bookfield viscometer at 25°C with No. 2 spindle rotating at 30 rpm. The results are shown in Fig. 13, from which one can see that $SP_2$ was less sensitive to pH than Kelzan in terms of viscosity.

In addition to the field of cosmetics, sanitary, paper diaper and greening desert, the following are examples of the applications in which the polysaccharide of the present invention may be put to use:

Food applications:

Thickeners, fillers, water retainers, texture improving agents, dietary foods;

Feedstuff applications:

Thickeners, fillers, water retainers, carrier entrapping;

Medical applications:

Immunoactivators, drug entrapping (e.g. capsules and tablets);

Biotechnological applications:

Immobilizers for use in bioreactors, etc., culture bases for microorganisms, plants and animal cells, supports (gels) for separation and purification;

Agricultural applications:

Capsules of slow release agents (e.g. agrichemicals), suspension stabilizers, emulsion stabilizers, improving adhesion, improving dust-ability, controlling the shape of liquid droplets;

Civil engineering:

Soil improving agents, soil water retainers, mud stabilizers, soil stabilizers;

Distribution

Drip absorbents for use in foods such as fish and meat;

Paper coating:

Improving the performance of coatings, preventing migration, preventing streaks, preventing pigment sedimentation, improving water retention;

Textile dyeing:

Preventing pigment sedimentation, preventing migration, improving dye fluidity, space dying;

Latices:

Emulsion stabilizers;

Cleaners:

Emulsion stabilizers, suspension stabilizers, anti-sagging agents, improving sprayability;

Suspension stabilizers:

Stabilizing $TiO_2$ suspensions, stabilizing the suspensions of starch slurry;

Foam stabilizers:

Foamed cement;

Improvement of polishing agents:

Buffing agents;

Paint improving agents:

Improving rheological properties.

## Claims

1. A polysaccharide producible by a microorganism of the genus Alcaligenes having the following properties:
   (A) sugar composition as determined by thin-layer chromatography, liquid chromatography and gas chromatography:
   the principal constituents are rhamnose, fucose, glucose, mannose and glucuronic acid which are present in a molar ratio of (1 - 10):(2 - 10):(4 - 20):(1):(1 - 5);
   (B) elemental analysis (wt%):

| | |
|---|---|
| C | 40 ± 4 |
| H | 6 ± 1 |
| O | 54 ± 5; |

   (C) carbonization point: 225 - 280 °C;
   (D) solubility:
   slightly soluble in water (neutral); soluble in alkalies; insoluble in methanol, ethanol and acetone;
   (E) UV absorption spectrum as shown in Fig 1;
   no absorption detected at 280 nm characteristic of proteins (peptides) or at 260 nm characteristic of nucleic acids; and
   (F) IR absorption spectrum as shown in Fig 2;
   having peaks at 800 - 1200 $cm^{-1}$, 1620 ± 20 $cm^{-1}$, 2950 ± 10 $cm^{-1}$ and 3400 ± 20 $cm^{-1}$.

2. A polysaccharide according to Claim 1 which contains 5 - 20 mol% glucuronic acid, with rhamnose, fucose, glucose and mannose being present in a molar ratio of (1 - 6):(3 - 5):(5 - 17):(1).

3. A polysaccharide according to Claim 1 which contains rhamnose, fucose, glucose, mannose and glucuronic acid in a molar ratio of (1 - 3):(3 - 5):(5 - 7):(1):(2 - 3).

4. A water absorbent, moisture absorbent or humectant and thickening agent which contains the polysaccharide of any of the Claims 1 to 3 as the main component.

5. A water absorbent, moisture absorbent or humectant and thickening agent according to Claim 4 which is essentially composed of a culture of an Alcaligenes microorganism or a treated product of said culture.

6. A water absorbent, moisture absorbent or humectant and thickening agent according Claim 5 wherein said Alcaligenes microorganism is Alcaligenes latus strain B-16 (FERM BP-2015).

7. A method of producing a polysaccharide according to any of the Claims 1 to 3 by cultivating an Alcaligenes microorganism under aeration with either a monosaccharide or disaccharide being selectively used as a carbon source in a culture medium so as to increase the yield of production of the polysaccharide.

8. A method of producing a water absorbent, moisture absorbent or humectant and thickening agent according to Claim 4 by cultivating an Alcaligenes microorganism under aeration with either a monosaccharide or disaccharide being selectively used as a carbon source in a culture medium so as to increase the yield of production of the water absorbent, moisture absorbent or humectant and thickening agent.

9. A method according to claim 7 or 8, wherein said Alcaligenes microorganism is Alcaligenes latus strain B-16 (FERM BP-2015).

**Patentansprüche**

1. Durch einen Mikroorganismus des Genus Alcaligenes herstellbares Polysaccharid, das die folgenden Eigenschaften besitzt:

(A) Durch Dünnschichtchromatographie, Flüssigphasenchromatographie und Gaschromatographie bestimmte Zusammensetzung aus Zuckern:
die Hauptbestandteile sind Rhamnose, Fucose, Glucose, Mannose und Glucuronsäure, die in einem Molverhältnis von (1 - 10) : (2 - 10) : (4 - 20) : (1) : (1 - 5) vorhanden sind;
(B) Elementaranalyse (Gew. %):

| | |
|---|---|
| C | 40 ± 4 |
| H | 6 ± 1 |
| O | 54 ± 5; |

(C) Verkokungspunkt 225 bis 280 ° C;
(D) Löslichkeit :
etwas löslich in Wasser (neutral), löslich in Alkalien, unlöslich in Methanol, Ethanol und Aceton;
(E) UV-Absorptionsspektrum wie in Figur 1 gezeigt;
keine Absorption bei 280 nm, die für Proteine (Peptide) charakteristisch ist oder bei 260 nm, die für Nucleinsäuren charakteristisch ist, und
(F) IR-Absorptionsspektrum wie in Figur 2 gezeigt, mit Maxima bei 800 bis 1200 $cm^{-1}$, 1620 ± 20 $cm^{-1}$, 2950 ± 10 $cm^{-1}$ und 3400 ± 20 $cm^{-1}$.

2. Polysaccharid nach Anspruch 1, das 5 bis 20 Mol % Glucuronsäure enthält, wobei Rhamnose, Fucose, Glucose und Mannose in einem Molverhältnis entsprechend
(1 - 6) : (3 - 5) : (5 - 17) : (1) vorhanden sind.

3. Polysaccharid nach Anspruch 1, welches Rhamnose, Fucose, Glucose, Mannose und Glucuronsäure in einem Molverhältnis entsprechend (1 - 3) : (3 - 5) : (5 - 7) : (1) : (2 - 3) enthält.

4. Wasserabsorptionsmittel, Feuchtigkeitsabsorptionsmittel oder Befeuchtungsmittel und Verdickungsmittel, welches das Polysaccharid nach einem der Ansprüche 1 bis 3 als Hauptbestandteil enthält.

5. Wasserabsorptionsmittel, Feuchtigkeitsabsorptionsmittel oder Befeuchtungsmittel und Verdickungsmittel nach Anspruch 4, welches im wesentlichen aus einer Kultur eines Alcaligenes-Mikroorganismus oder einem Aufarbeitungsprodukt dieser Kultur besteht.

6. Wasserabsorptionsmittel, Feuchtigkeitsabsorptionsmittel oder Befeuchtungsmittel und Verdickungsmittel nach Anspruch 5, wobei der Alcaligenes-Mikroorganismus Alcaligenes latus Stamm B-16 (FEPM BP-2015) ist.

7. Verfahren zur Herstellung eines Polysaccharids nach einem der Ansprüche 1 bis 3 durch Züchten eines Alcaligenes-Mikroorganismus unter Belüftung und unter selektiver Verwendung entweder eines Monosaccharids oder Disaccharids als Kohlenstoffquelle in einem Kulturmedium, um so die Produktionsausbeute des Polysaccharids zu erhöhen.

8. Verfahren zur Herstellung eines Wasserabsorptionsmittels, Feuchtigkeitsabsorptionsmittels oder Befeuchtungsmittels und Verdickungsmittels nach Anspruch 4 durch Züchten eines Alcaligenes-Mikroorganismus unter Belüftung und unter selektiver Verwendung entweder eines Monosaccharids oder Disaccharids als Kohlenstoffquelle in einem Kulturmedium, um so die Produktionsausbeute des Wasserabsorptionsmittels, Feuchtigkeitsabsorptionsmittels oder Befeuchtungsmittels und Verdickungsmittels zu erhöhen.

9. Verfahren nach Anspruch 7 oder 8, wobei der Alcaligenes-Mikroorganismus Alcaligenes latus Stamm B-16 (FEBM-2015) ist.

## Revendications

1. Polysaccharide productible par un microorganisme du genre *Alcaligenes*, ayant les propriétés suivantes :
   (A) composition en sucres telle que déterminée par chromatographie en couche mince, chromatographie en phase liquide et chromatographie en phase gazeuse :
   les principaux constituants sont le rhamnose, le fucose, le glucose, le mannose et l'acide glucuronique qui sont présents en un rapport molaire de (1 à 10):(2 à 10):(4 à 20):(1):(1 à 5) ;
   (B) analyse élémentaire (% en poids) :

| | |
|---|---|
| C | 40 ± 4 |
| H | 6 ± 1 |
| O | 54 ± 5 ; |

   (C) point de carbonisation : 225-280 °C ;
   (D) solubilité :
   légèrement soluble dans l'eau (neutre) ; soluble dans les alcalis ; insoluble dans le méthanol, l'éthanol et l'acétone ;
   (E) spectre d'absorption UV tel que montré sur la Figure 1 :
   aucune absorption détectée à 280 nm, caractéristique des protéines (peptides), ni à 260 nm, caractéristique des acides nucléiques ; et
   (F) spectre d'absorption IR tel que montré sur la Figure 2 :
   ayant des pics à 800-1200 $cm^{-1}$, 1620 ± 20 $cm^{-1}$, 2950 ± 10 $cm^{-1}$ et 3400 ± 20 $cm^{-1}$.

2. Polysaccharide selon la revendication 1, qui contient 5 à 20 mol % d'acide glucuronique, le rhamnose, le fucose, le glucose et le mannose étant présents en un rapport molaire de (1 à 6):(3 à 5):(5 à 17):(1).

3. Polysaccharide selon la revendication 1, qui contient du rhamnose, du fucose, du glucose, du mannose et de l'acide glucuronique en un rapport molaire de (1 à 3):(3 à 5):(5 à 7):(1 :(2 à 3).

4. Agent absorbant d'eau, absorbant d'humidité ou humectant et épaississant, qui contient le polysaccharide de l'une quelconque des revendications 1 à 3 comme principal composant.

5. Agent absorbant d'eau, absorbant d'humidité ou humectant et épaississant selon la revendication 4, qui est essentiellement constitué d'un produit de culture d'un microorganisme du genre *Alcaligenes* ou d'un produit traité dudit produit de culture.

6. Agent absorbant d'eau, absorbant d'humidité ou humectant et épaississant selon la revendication 5, dans lequel ledit microorganisme du genre *Alcaligenes* est la souche *Alcaligenes latus* B-16 (FERM BP-2015).

7. Procédé de production d'un polysaccharide selon l'une quelconque des revendications 1 à 3, par culture d'un microorganisme du genre *Alcaligenes* sous aération en utilisant sélectivement un monosaccharide ou un disaccharide comme source de carbone dans un milieu de culture de manière à accroître le rendement de production du polysaccharide.

8. Procédé de production d'un agent absorbant d'eau, absorbant d'humidité ou humectant et épaississant selon la revendication 4, par culture d'un microorganisme du genre *Alcaligenes* sous aération en utilisant sélectivement un monosaccharide ou un disaccharide comme source de carbone dans un milieu de culture de manière à accroître le rendement de production de l'agent absorbant d'eau, absorbant d'humidité ou humectant et épaississant.

9. Procédé selon la revendication 7 ou 8, dans lequel ledit microorganisme du genre *Alcaligenes* est la souche *Alcaligenes latus* B-16 (FERM BP-2015).

Fig. 1

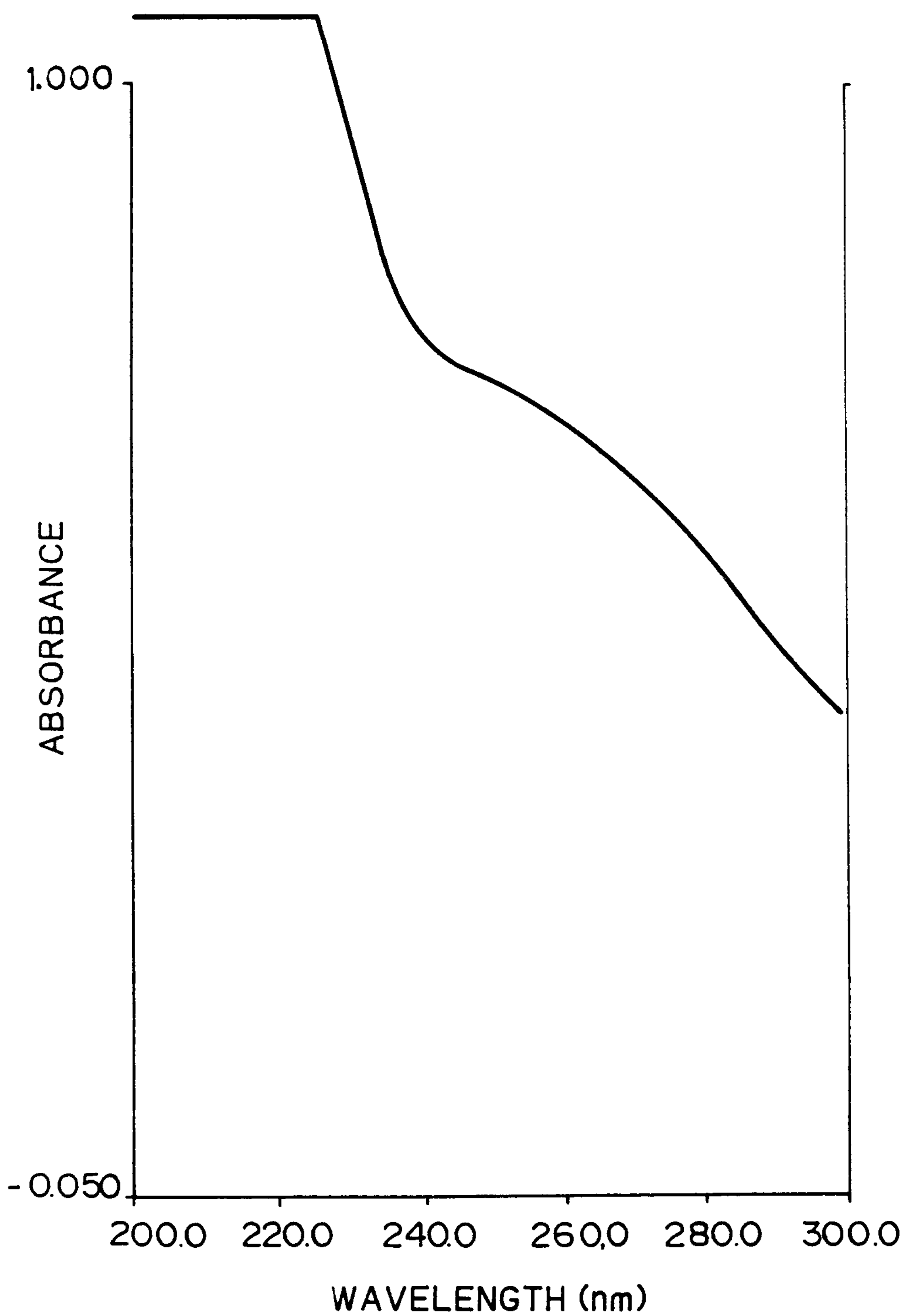
1.000
-0.050
ABSORBANCE
200.0
220.0
240.0
260.0
280.0
300.0
WAVELENGTH (nm)

Fig.2

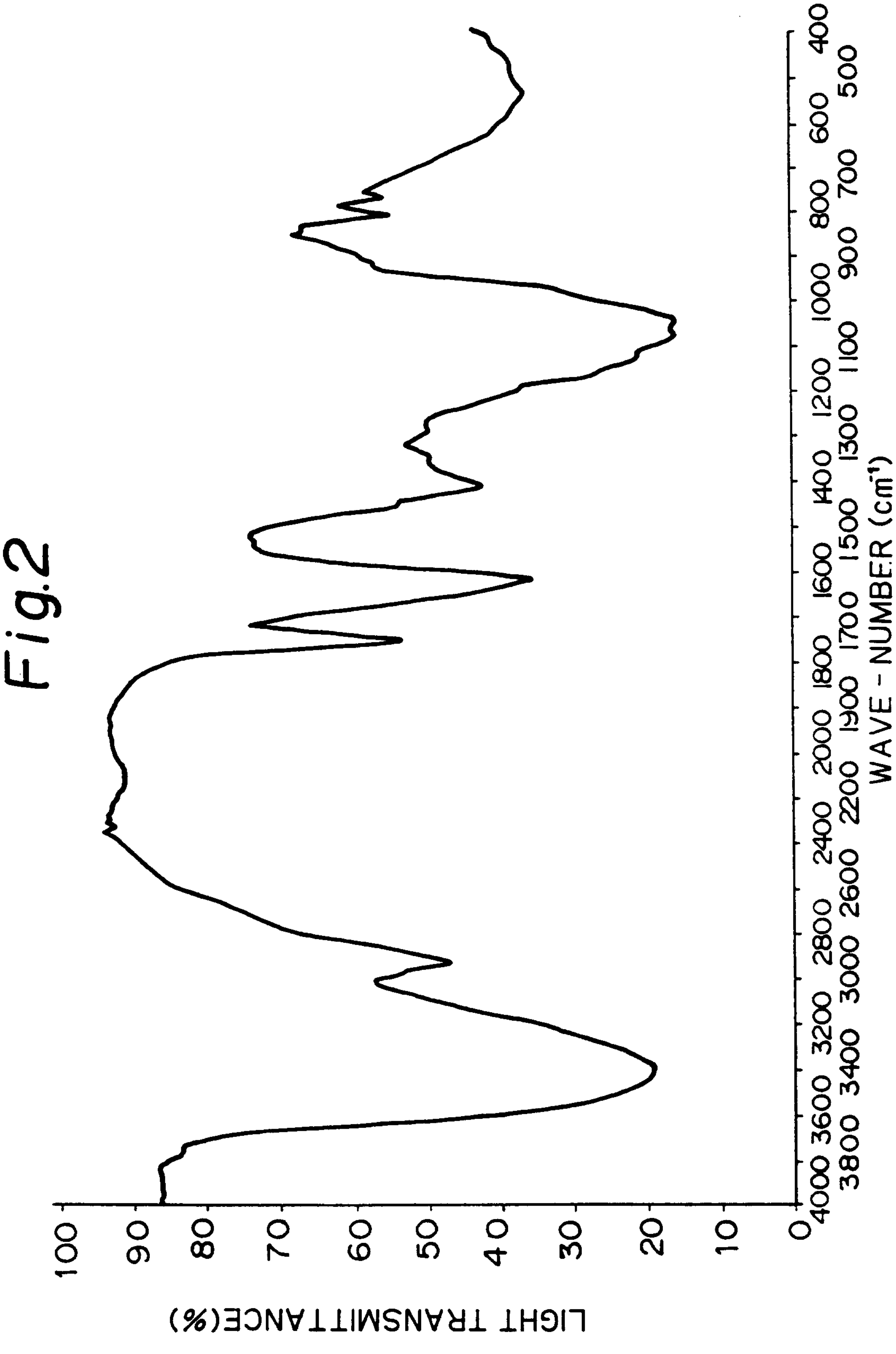
LIGHT TRANSMITTANCE(%)
100
90
80
70
60
50
40
30
20
10
0
4000 3800 3600 3400 3200 3000 2800 2600 2400 2200 2000 1900 1800 1700 1600 1500 1400 1300 1200 1100 1000 900 800 700 600 500 400
WAVE - NUMBER (cm⁻¹)

Fig. 3

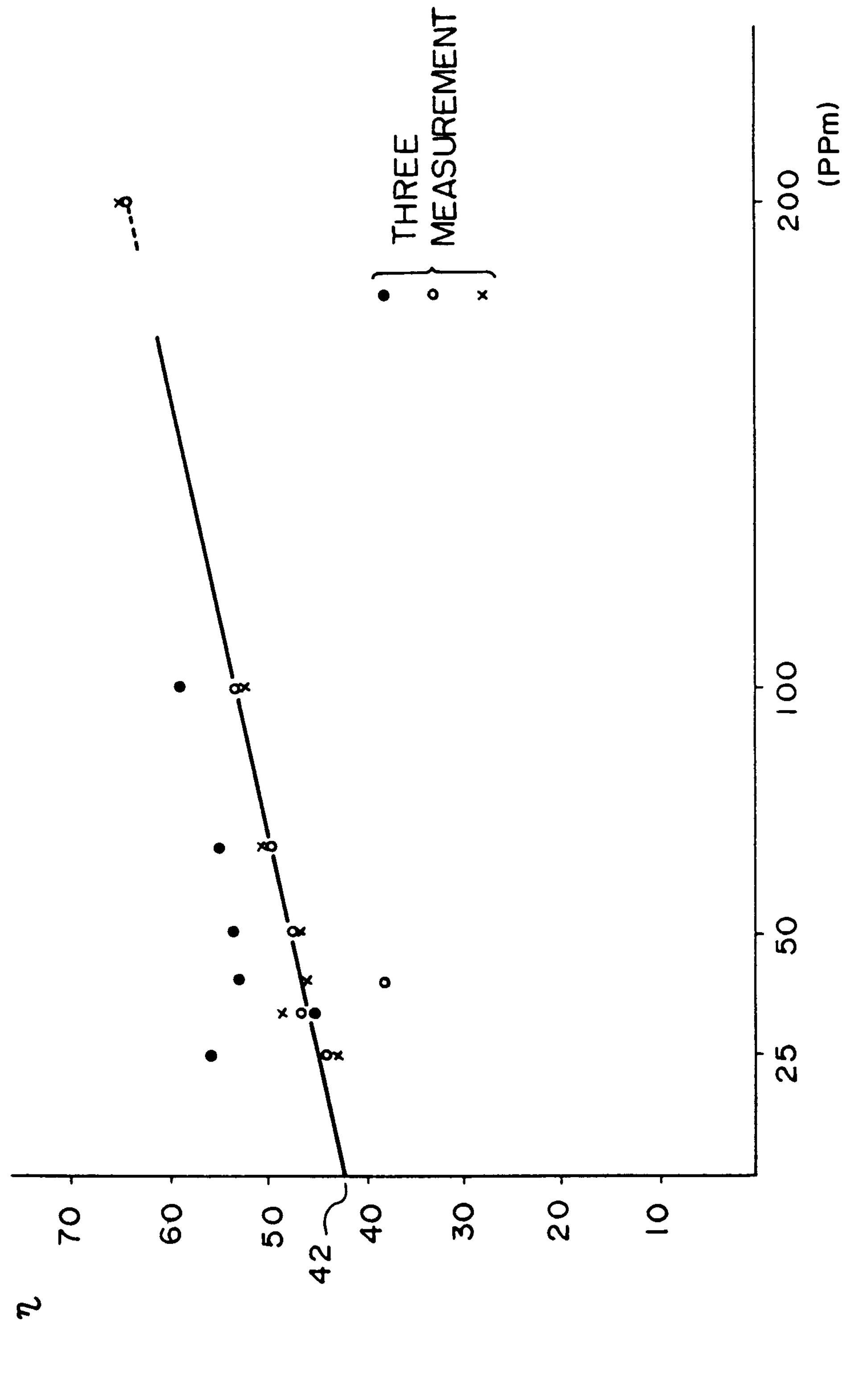

Fig. 4-A

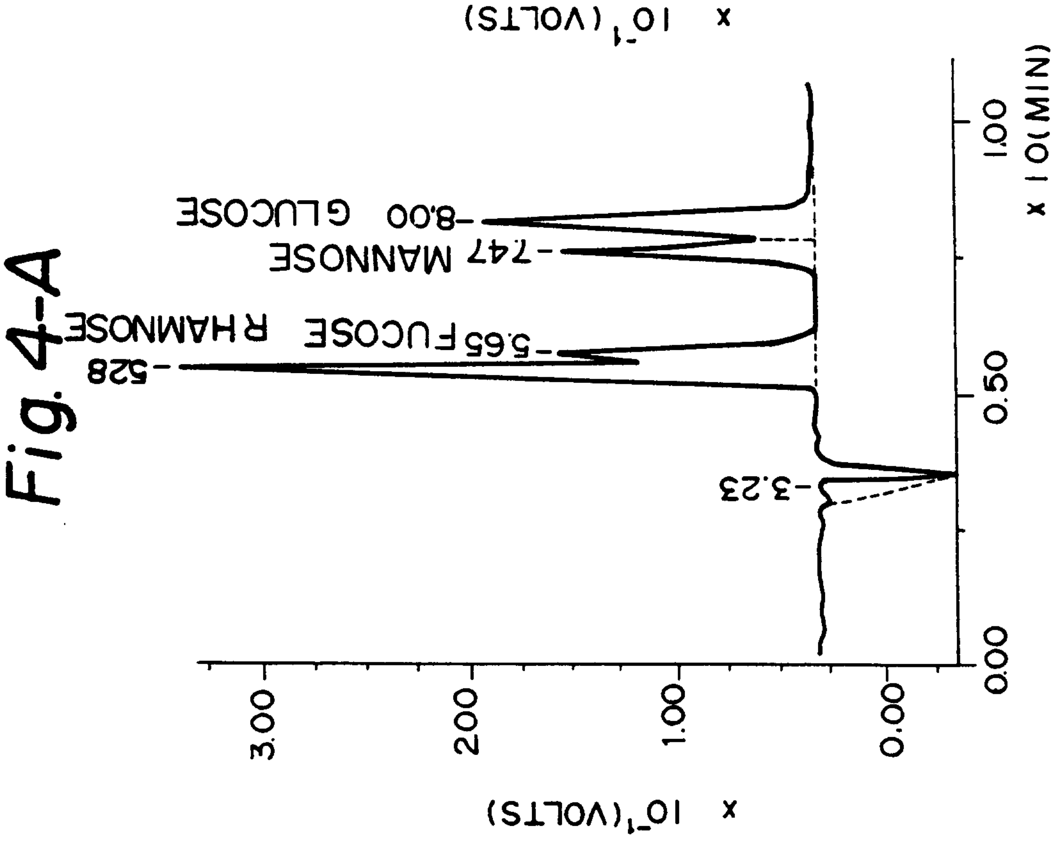
x 10⁻¹(VOLTS)
3.00
2.00
1.00
0.00
0.00
0.50
1.00
x 10(MIN)
-3.23
-528
RHAMNOSE
-5.65 FUCOSE
-7.47 MANNOSE
-8.00 GLUCOSE

Fig. 4-B

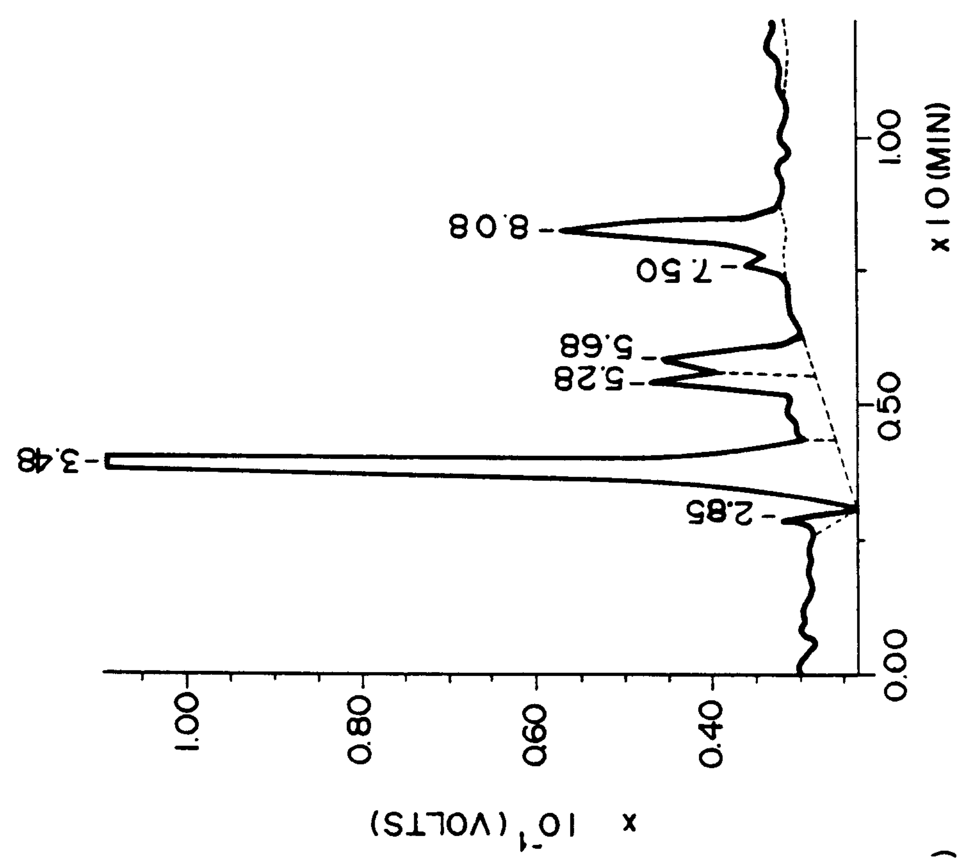
x 10⁻¹(VOLTS)
1.00
0.80
0.60
0.40
0.00
0.50
1.00
x 10(MIN)
-2.85
-3.48
-5.28
-5.68
-7.50
-8.08

Fig. 4-D

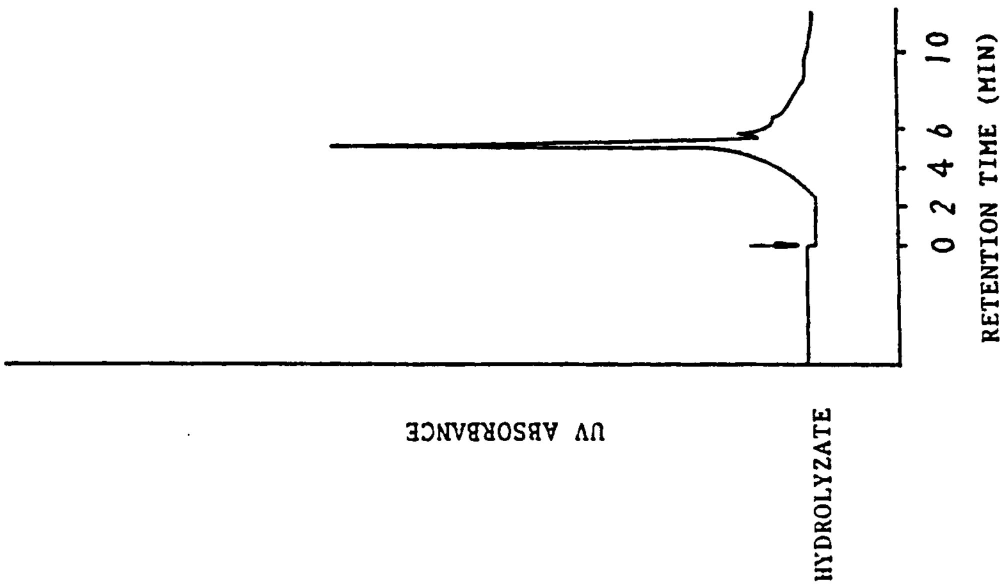
UV ABSORBANCE
HYDROLYZATE
0 2 4 6 10
RETENTION TIME (MIN)

Fig. 4-C

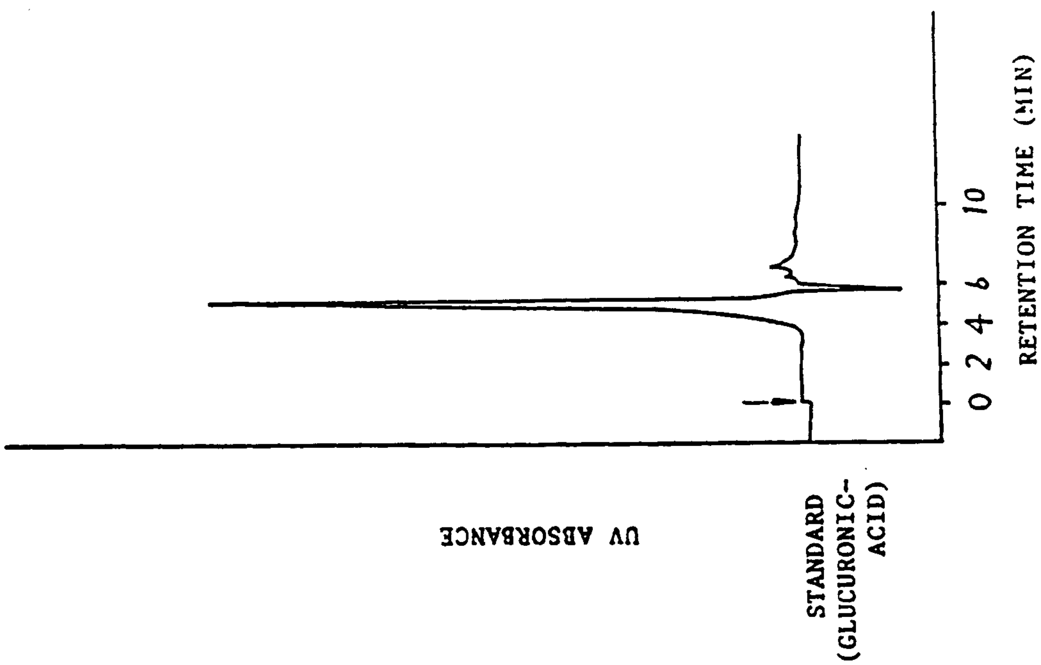
UV ABSORBANCE
STANDARD
(GLUCURONIC-
ACID)
0 2 4 6 10
RETENTION TIME (MIN)

Fig.5-B

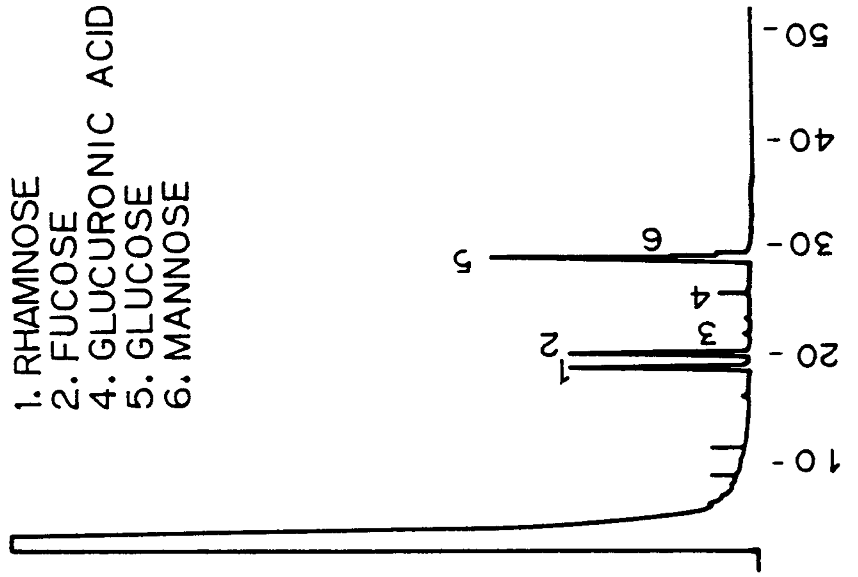
1. RHAMNOSE
2. FUCOSE
4. GLUCURONIC ACID
5. GLUCOSE
6. MANNOSE
1
2
3
4
5
6
10
20
30
40
50

Fig. 5-A

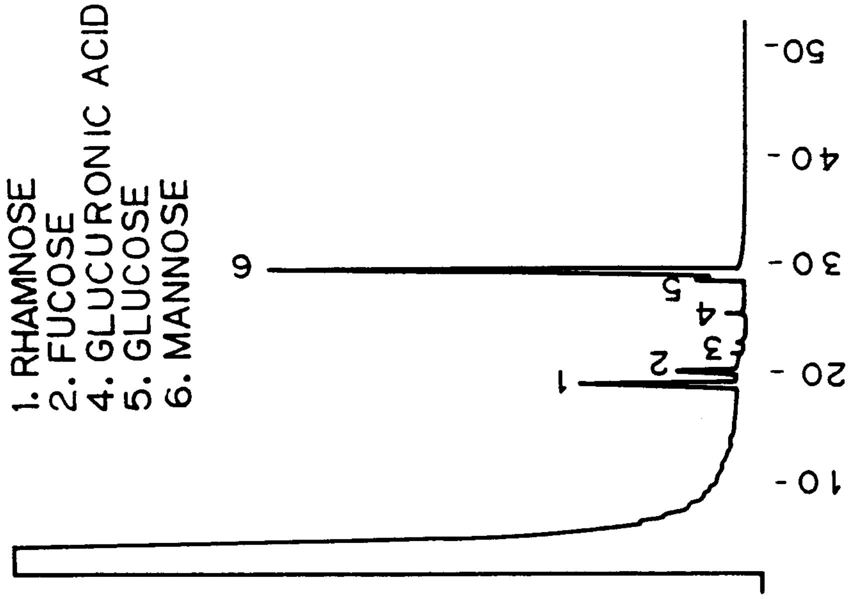
1. RHAMNOSE
2. FUCOSE
4. GLUCURONIC ACID
5. GLUCOSE
6. MANNOSE
1
2
3
4
5
6
10
20
30
40
50

Fig.6-1A

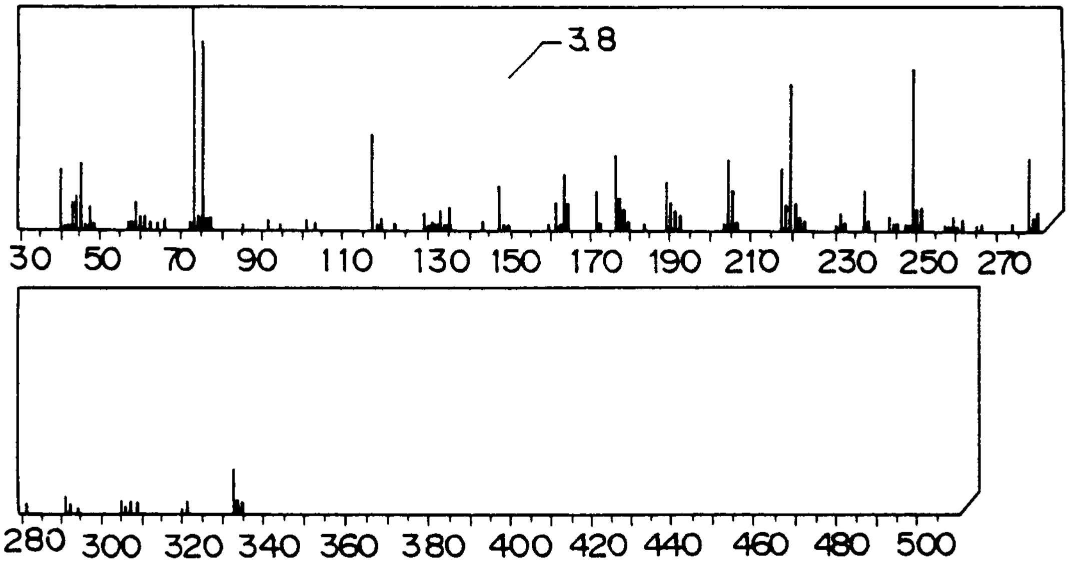


Fig.6-1B

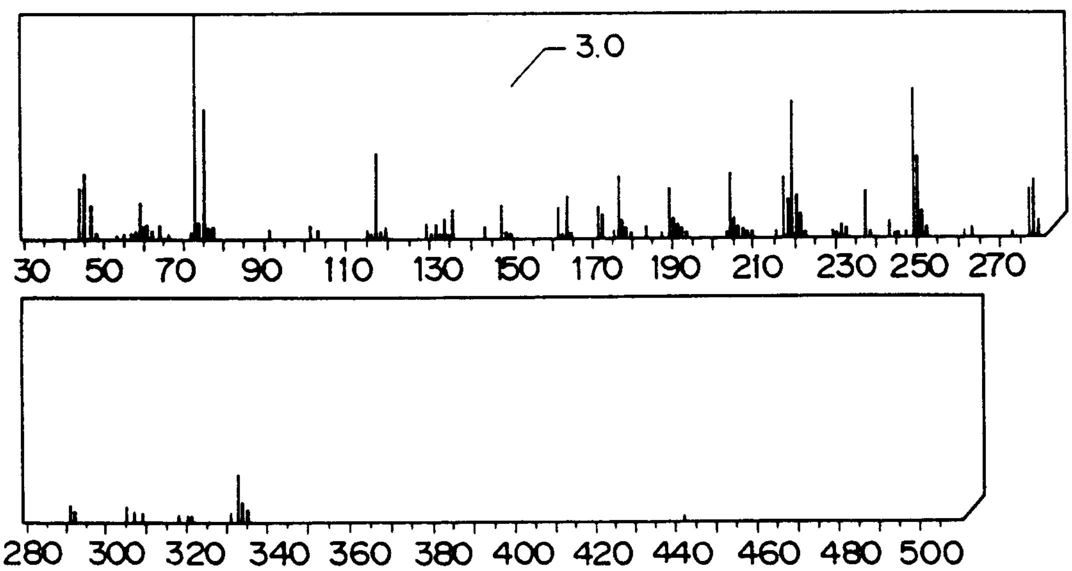

Fig.6-2A

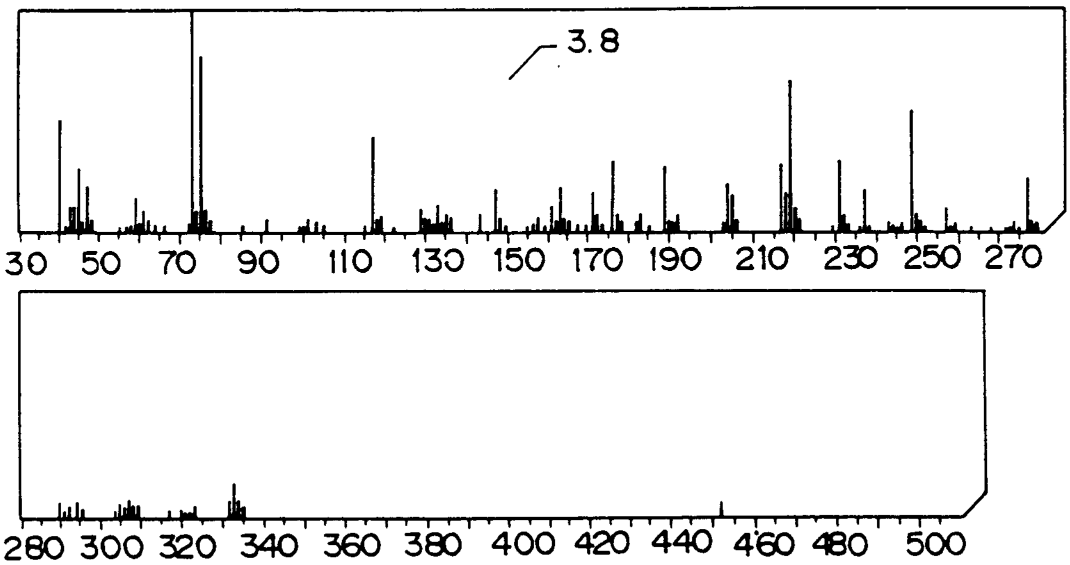


Fig.6-2B

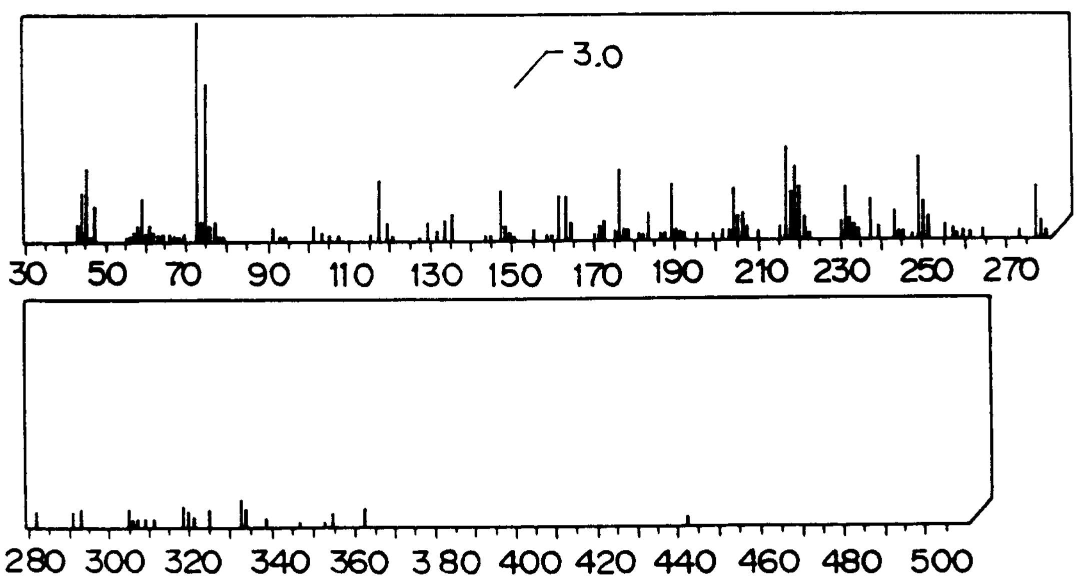

Fig. 6-3A

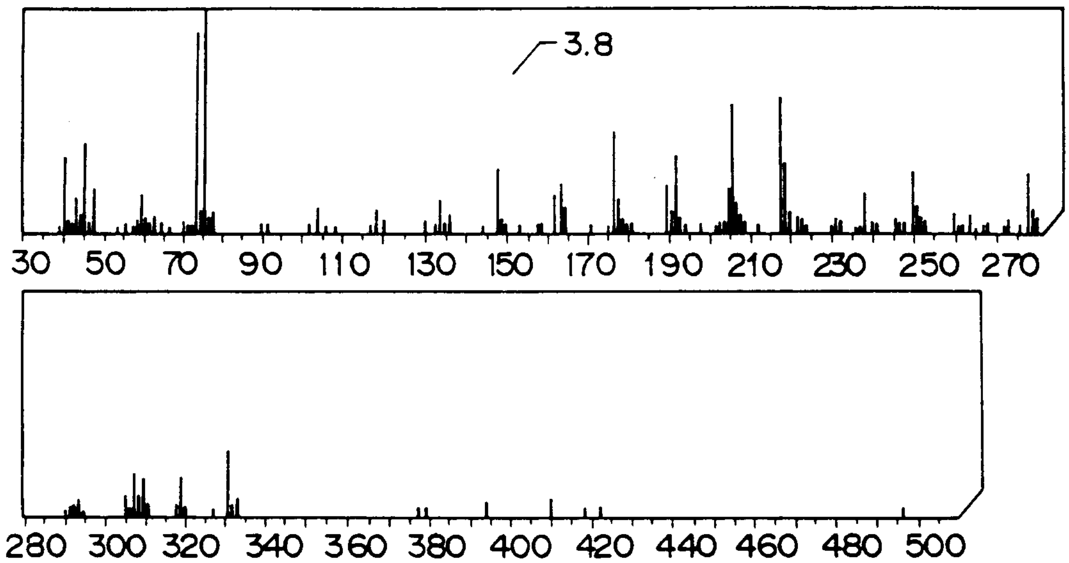


Fig. 6-3B

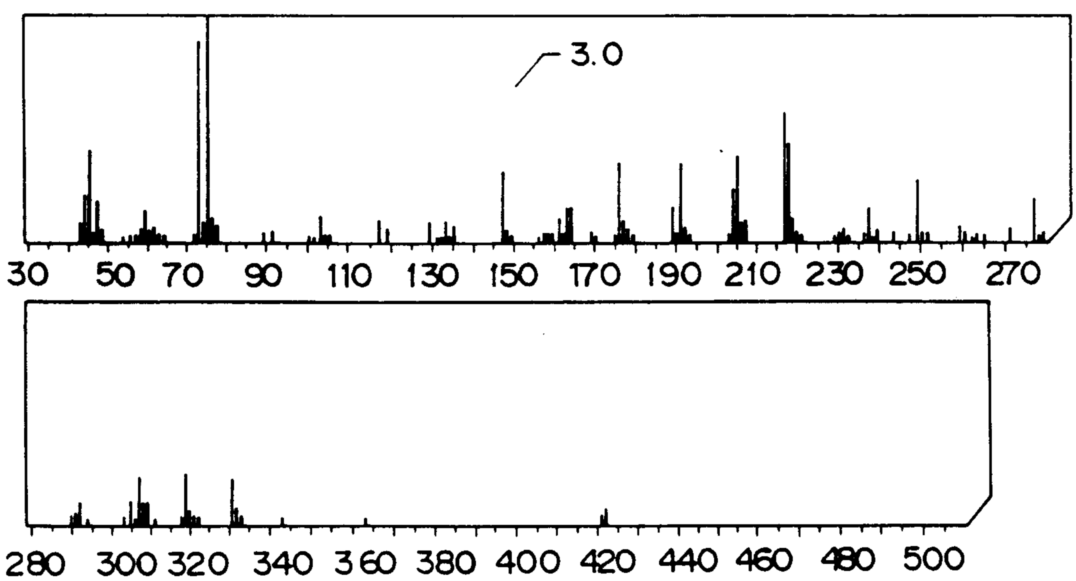

Fig.6-4A

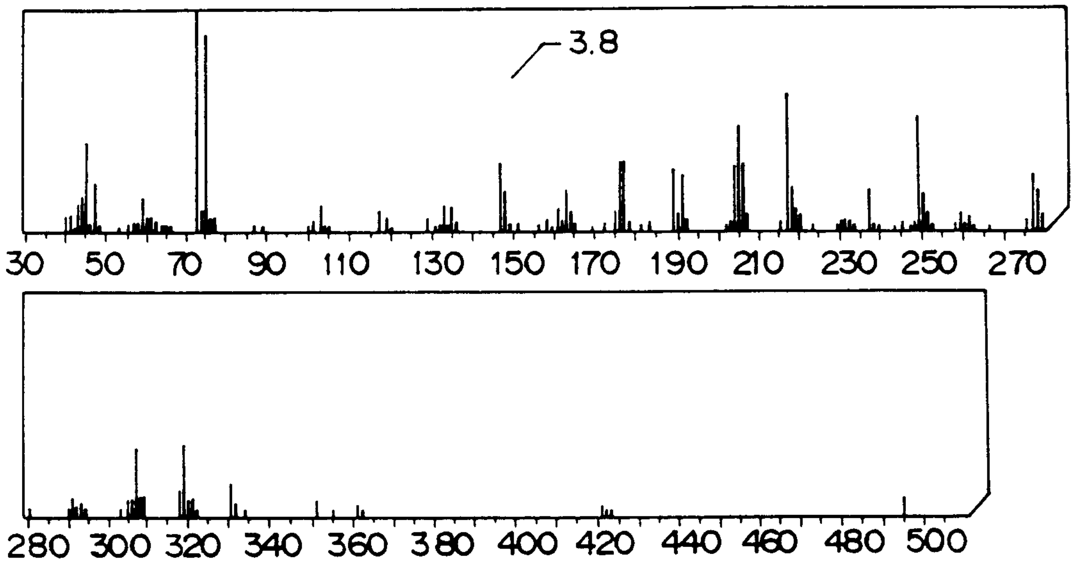


Fig.6-4B

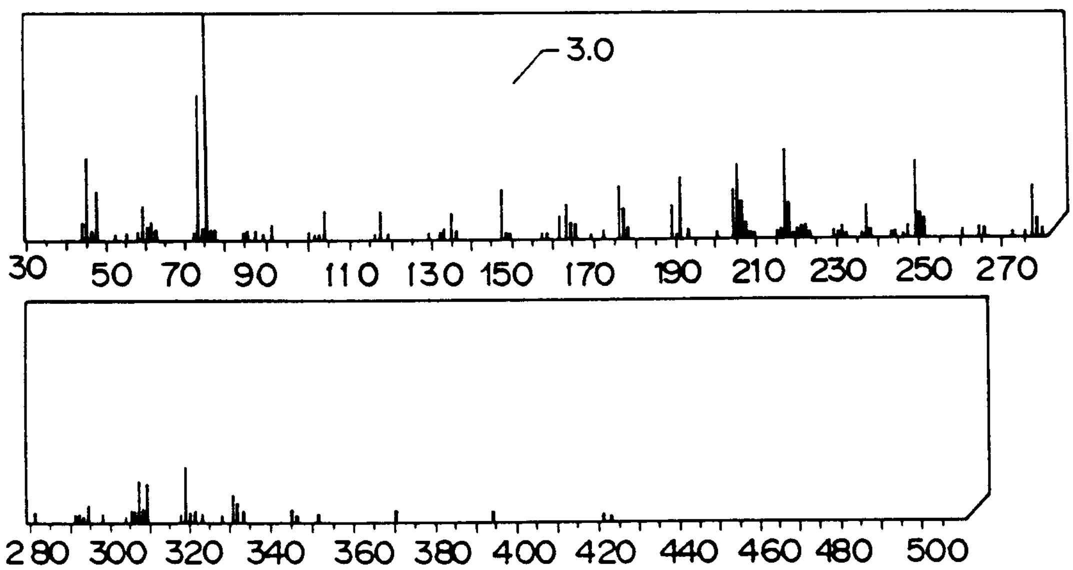

Fig. 7-B

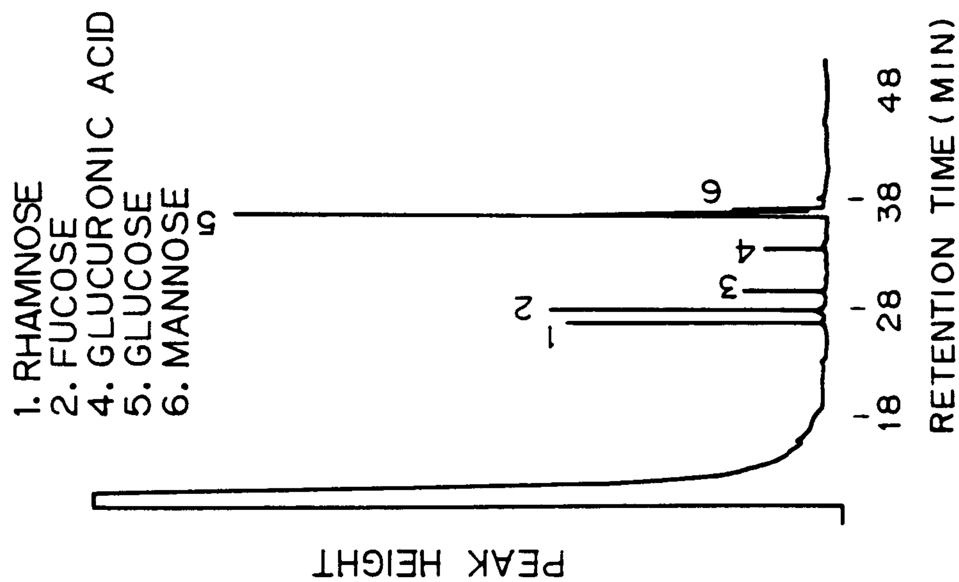
1. RHAMNOSE
2. FUCOSE
4. GLUCURONIC ACID
5. GLUCOSE
6. MANNOSE
1
2
3
4
5
6
PEAK HEIGHT
18
28
38
48
RETENTION TIME (MIN)

Fig. 7-A

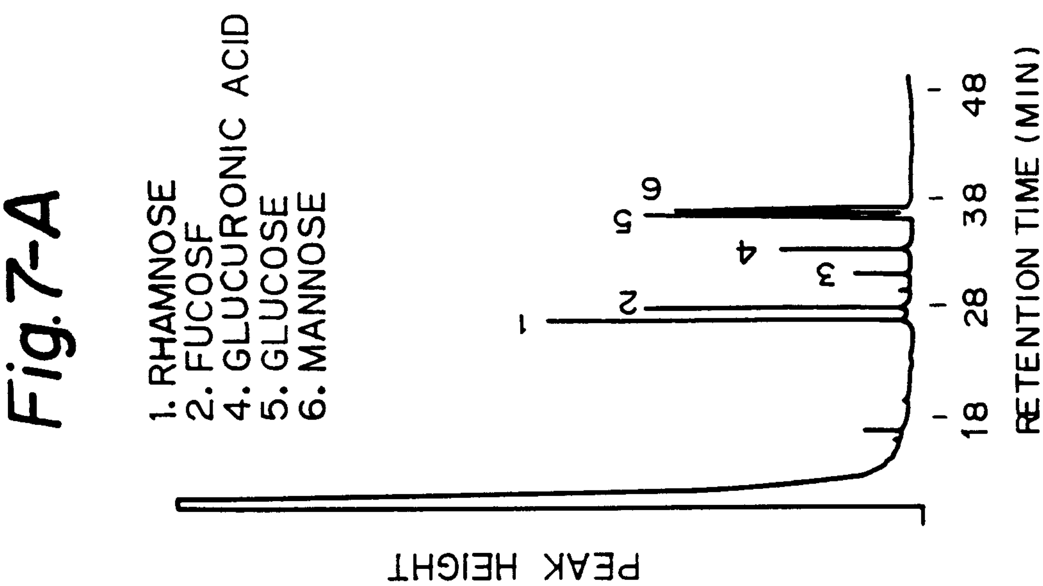
1. RHAMNOSE
2. FUCOSF
4. GLUCURONIC ACID
5. GLUCOSE
6. MANNOSE
1
2
3
4
5
6
PEAK HEIGHT
18
28
38
48
RETENTION TIME (MIN)

Fig. 8

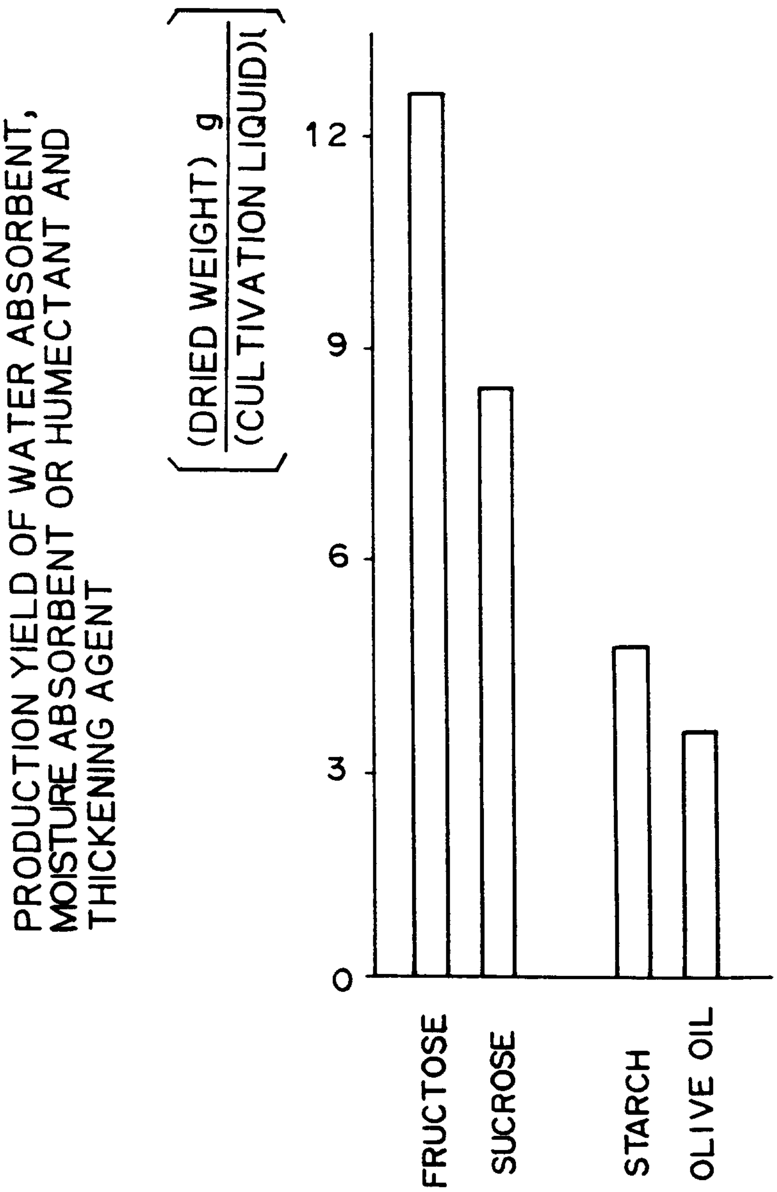
PRODUCTION YIELD OF WATER ABSORBENT, MOISTURE ABSORBENT OR HUMECTANT AND THICKENING AGENT
(DRIED WEIGHT) g / (CULTIVATION LIQUID) l
0
3
6
9
12
FRUCTOSE
SUCROSE
STARCH
OLIVE OIL

Fig. 9

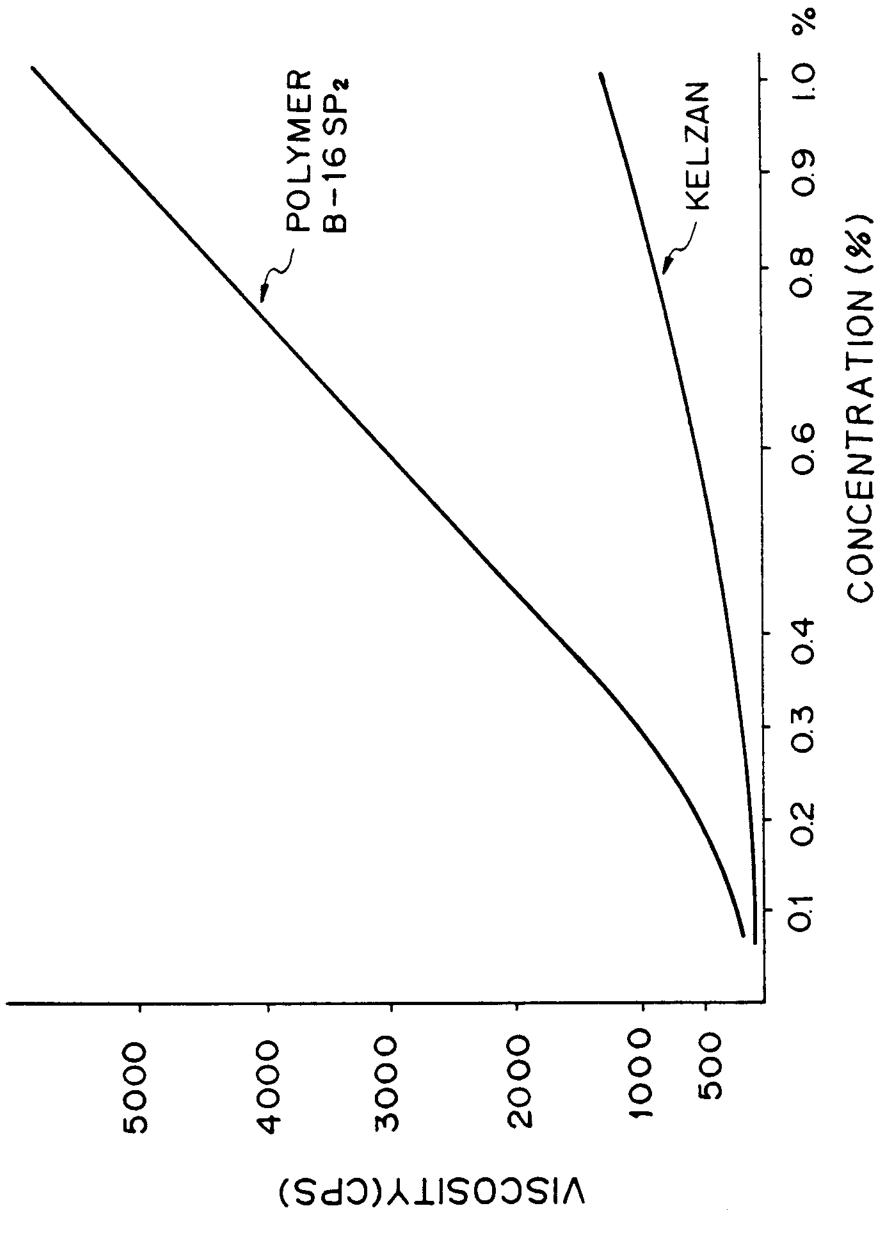

POLYMER
B-16 $SP_2$
KELZAN
VISCOSITY(CPS)
5000
4000
3000
2000
1000
500
0.1
0.2
0.3
0.4
0.6
0.8
0.9
1.0
%
CONCENTRATION (%)

Fig.10-A

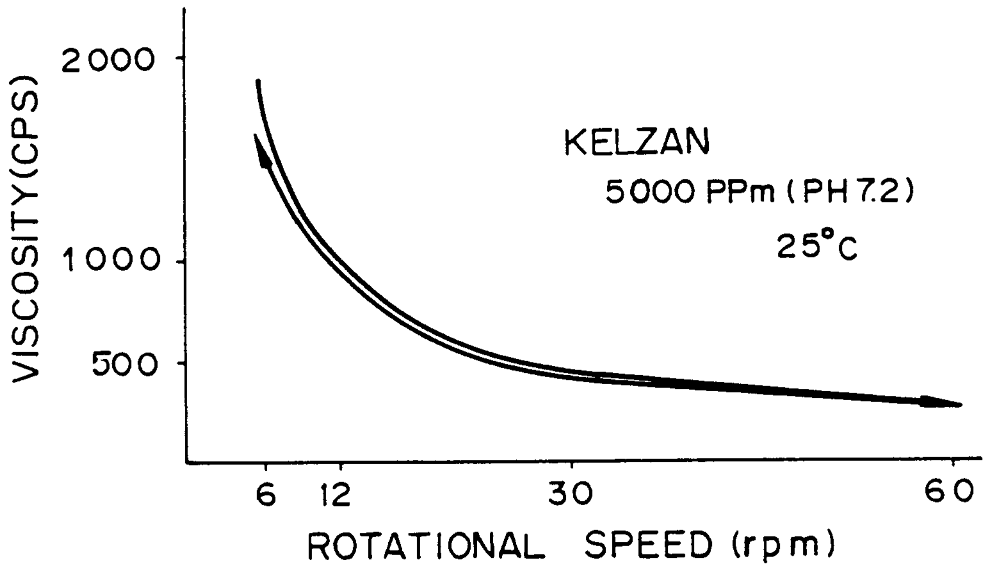


Fig.10-B

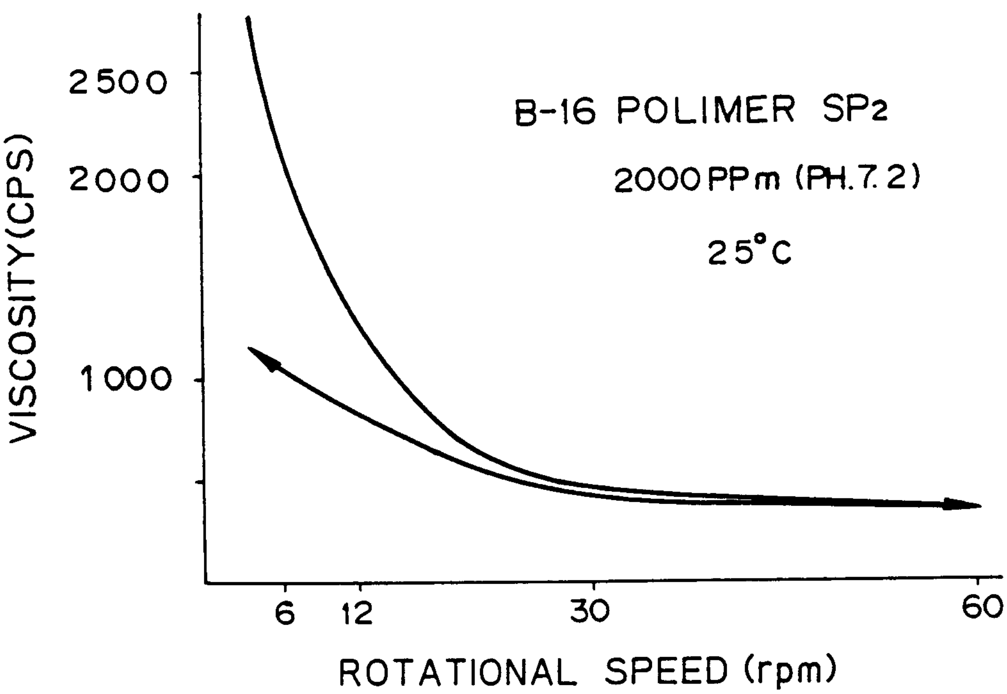

Fig.11

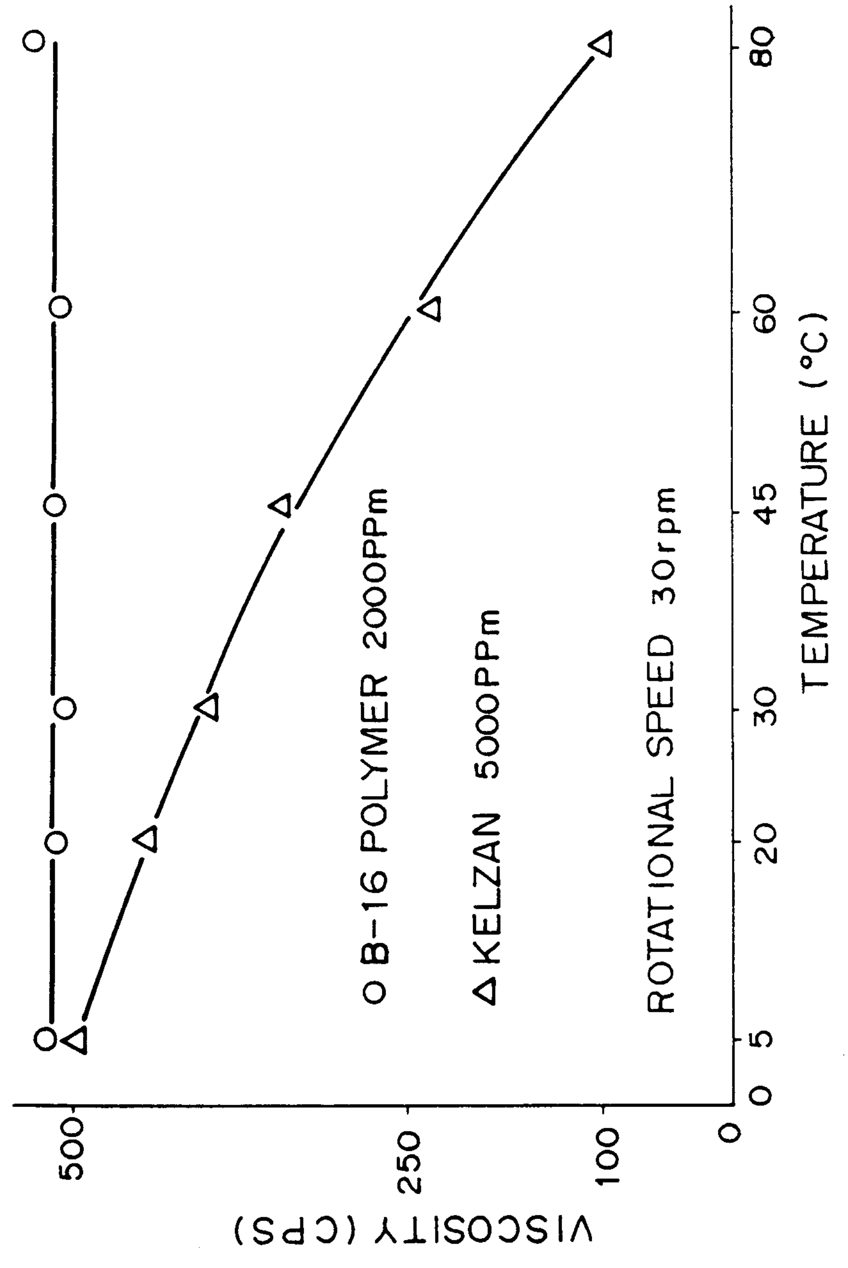
O B-16 POLYMER 2000PPm
Δ KELZAN 5000PPm
ROTATIONAL SPEED 30rpm
VISCOSITY (CPS)
500
250
100
0
0
5
20
30
45
60
80
TEMPERATURE (°C)

Fig.12

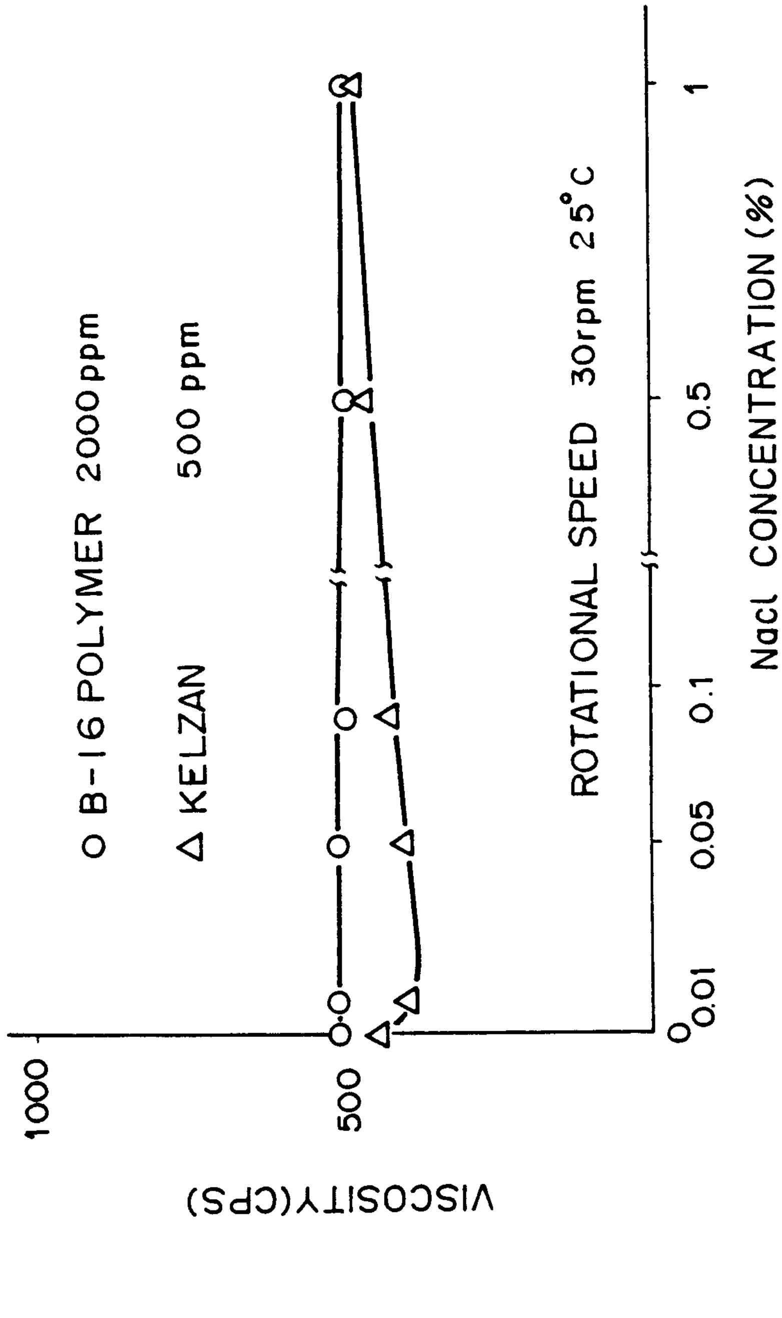
○ B-16 POLYMER 2000 ppm
△ KELZAN 500 ppm
ROTATIONAL SPEED 30rpm 25°C
VISCOSITY(CPS)
1000
500
0
0.01
0.05
0.1
0.5
1
Nacl CONCENTRATION (%)

Fig.13

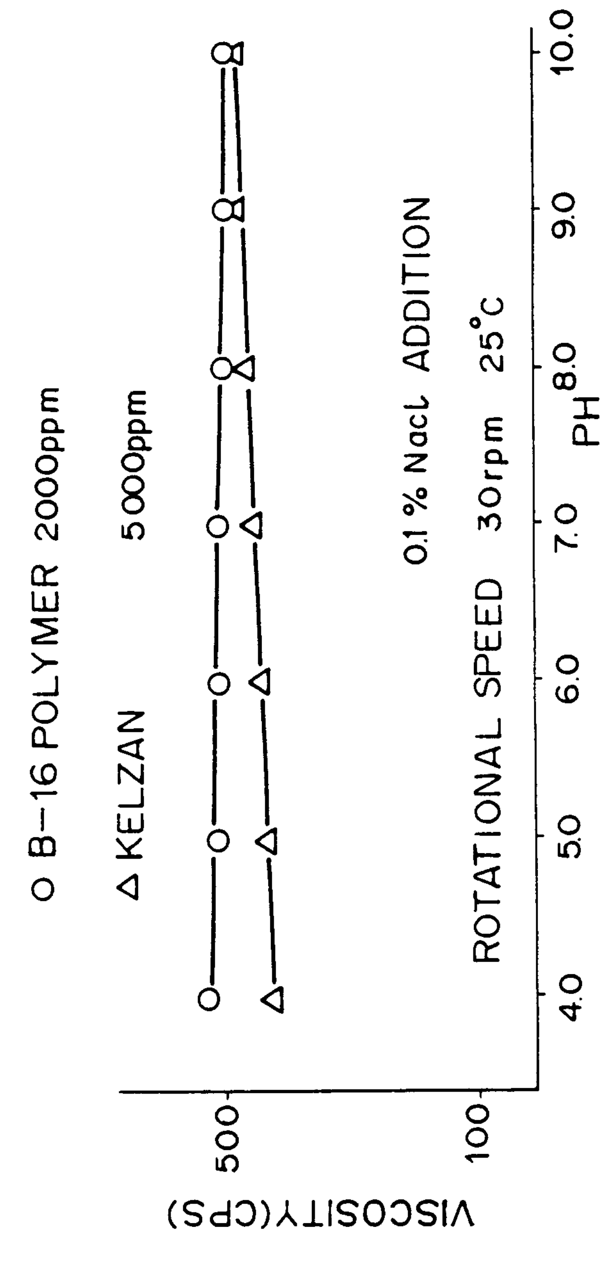

○ B-16 POLYMER 2000ppm
△ KELZAN 5000ppm
0.1 % Nacl ADDITION
ROTATIONAL SPEED 30rpm 25°C
VISCOSITY(CPS)
500
100
PH
4.0
5.0
6.0
7.0
8.0
9.0
10.0